# EUROPEAN PATENT SPECIFICATION

(11) **EP 1 680 442 B1**
(45) Date of publication and mention of the grant of the patent: **13.07.2011**
(21) Application number: 04796370.7
(22) Date of filing: 22.10.2004
(51) Int. Cl.: G01N 33/00

(54) **PIF PEPTIDES BIOLOGIC ACTIVITIES**
BIOLOGISCHE WIRKUNGEN VON PIF-PEPTIDEN
ACTIVITES BIOLOGIQUES DE PEPTIDES PIF

(30) Priority: 22.10.2003 US 513370 P
(43) Date of publication of application: 19.07.2006
(73) Proprietor: Bioincept, LLC., Cherry Hill, NJ 08003-3157 (US)
(72) Inventor: BARNEA, Eytan R., Cherry Hill, NJ 08003-3157 (US)
(74) Representative: Ouzman, Beverley Nicola Claire
(86) International application number: PCT/US2004/035382
(87) International publication number: WO 2005/040196

(56) References cited:
- WO-A-03/004601
- WO-A-2004/053086
- US-A- 5 646 003
- COULAM C B ET AL: "PREIMPLANTATION FACTOR (PIF) PREDICTS SUBSEQUENT PREGNANCY LOSS" AMERICAN JOURNAL OF REPRODUCTIVE IMMUNOLOGY, MUNKSGAARD INTERNATIONAL PUBLISHERS, COPENHAGEN, DK, vol. 34, no. 2, August 1995 (1995-08), pages 88-92, XP001096382 ISSN: 1046-7408
- GONZALEZ ET AL: "Preimplantation factor (PIF) may modulate maternal cellular immunity (CD2)" AMERICAN JOURNAL OF REPRODUCTIVE IMMUNOLOGY, MUNKSGAARD INTERNATIONAL PUBLISHERS, COPENHAGEN, DK, vol. 46, no. 1, July 2001 (2001-07), pages 68-69, XP002966956 ISSN: 1046-7408
- GONZALEZ ET AL.: 'PREIMPLANTATION FACTORS (PIF) EMBRYO-DERIVED IMMUNOMODULATORY PEPTIDES: POSSIBLE IMPLICATIONS FOR MATERNAL RECOGNITION AND ALLOGRAFT TOLERANCE' AME.J. OF REPROD IMMUN. vol. 47, no. 6, 2002, XP003000084
- ROUSSEV ET AL.: 'Development and Validation of an assay for measuring preimplantation factor (PIF) of embryonal arigin' AMERICAN JOURNAL OF REPRODUCTIVE IMMUNOLOGY vol. 35, 1996, pages 281 - 287, XP008067941
- ROUSSEV ET AL.: 'A novel bioassay for detection of preimplantation factor (PIF)' AMERICAN JOURNAL OF REPRODUCTIVE IMMUNOLOGY vol. 33, 1995, pages 68 - 73, XP002966955

## Description

### BACKGROUND

Fertilization requires the proper interaction between the egg and sperm. Such a targeted event, even under natural conditions, is random, and hence the genetic make up of the nascent genome is unpredictable. The impending pregnancy has to prepare the maternal environment towards acceptance of a semi- or even a total allograft. This preparation can be divided to four distinct phases; the first is the pre-fertilization period, the second is the fertilization/post fertilization, the third phase is trophoblast development, and the final fourth phase is the implantation period.

The first phase, which is the pre-fertilization period, takes place during follicular development. The egg is surrounded by the cumulus oophorus and it is bathed in the rich follicular fluid. This fluid has some immune suppressive activity that may facilitate the fertilization process as well as post-fertilization development. This immune suppressive activity is required, due to the fact that shortly after fertilization, expression of foreign antigens, caused by the sperm, may be present. This mechanism, however, is not a necessary requirement. In cases of *in vitro* fertilization, no such fluid is available, and fertilization takes place without difficulty in an artificial environment. Moreover, women who have no ovaries can get pregnant by donor embryo transfer.

The next phase is the fertilization/post fertilization process. Once the sperm penetrates the egg it becomes "non visible" by the maternal environment. During the process of egg and sperm head fusion, as long as the egg surface membrane does not change its characteristics and become recognizable by the maternal immune system, no immune reaction would be expected. To safeguard the fertilized egg, it is rapidly surrounded by the zona pellucida, which is a hard and impenetrable shell designed to ward off immune cells. A further protection is due to the presence of the maternal cumulus cells. Those cells may further prevent direct access of immune cells to the embryo. The cumulus cells persist only for the first few days after fertilization because they facilitate the fallopian tube's cilia to propagate the zygote towards the uterus. Following fertilization, it is not excluded that small proteins derived from - the maternal environment, such as cytokines, will reach the zygote and early embryo. So far, there has been no evidence for such an occurrence. Following the few initial embryonic cell divisions, to the eight-cell stage, the trophoblast phase is initiated.

The trophoblast phase that occurs by the sixteen-cell stage leads to embryoblast and trophoblast differentiation. While the trophoblast's genome is principally paternally derived, the embryoblast's genome is principally maternally derived. However, since the zona pellucida still surrounds the embryo, it provides a major protection against maternal immune onslaught. Therefore, it appears that the early embryo, during the peri-implantation phase, is rather well protected from maternal immune system. This is despite the fact that the embryo is a semi-antigen. This period in *in vitro* fertilization/embryo transfer (IVF/ET) procedures does not occur. Consequently, the development of maternal tolerance remains permissive until implantation, which is where direct embryo/maternal contact become a necessary prerequisite.

The final preparation phase is implantation, which occurs when the embryo reaches the uterus and intimate maternal contact is initiated. During implantation the zona pellucida opens, and the trophoblast cells are extruded. This is the time that the embryo is most vulnerable. The embryo is not yet attached to the maternal surface, and it is still exposed to endometrial maternal immune cells as well as potentially hostile cytokines. Of all phases of reproduction, the implantation phase is the most crucial. Specifically, in the case of embryo transfer, following IVF, the embryo has to sojourn in the endometrial cavity for 4-5 days until the maternal organism will accept it. This is a period of endometrial priming by embryonic signaling that leads to maternal tolerance, which is the pre-requisite for successful pregnancy.

Mammalian reproduction was the last to evolve and it required a major shift in the immune system. This is because it allowed a sperm, which might be regarded as a parasite, to invade the maternal organism, and impose, in part, its own genome expression. This suggests that the embryo must have an active role in allowing the initiation of pregnancy. This suggestion is supported by the following observations:
(i) Donor embryos can implant without difficulty, therefore sharing of maternal antigens is not required;
(ii) The site of embryo implantation is not obligatory, although the uterus is preferred. Occasionally, implantation can be found in the fallopian tubes, ovaries, and even inside the abdominal cavity;
(iii) Under certain circumstances, embryos from one species can be implanted and delivered by another species. Genetic mismatch also does not prevent a successful reproduction (*i.e*.: mule);
(iv) Only viable embryos will implant. Therefore, the implantation is an active act that requires a passive accommodation by the maternal recipient, upon which the embryo can impose its will, in order for the pregnancy to develop;
(v) Sick mammals can also get pregnant, which indicates that the maternal organism does not need to be in good health in order for pregnancy to initiate. This reiterates the passive role of the maternal organism and supports a specific embryo effect;
(vi) The chances of multiple embryos to implant are higher than that of a single embryo. Consequently, an enhanced embryo derived signaling is likely to lead to maternal acceptance; and
(vii) Although a window of opportunity for implantation does exist, it is not strict. Therefore the embryo can implant in less than favorable endometrium, as well.

In conclusion, it appears that the embryo, to a large degree, controls it own destiny. This destiny is irrespective of timing in cycle, site of implantation, the sharing of genes, species, or the health of the mother.

Early work on pregnancy suggested that shortly after fertilization, certain changes that favor tolerance take place in the maternal environment. Those changes were believed to be due to early pregnancy factor (EPF) and platelet activating factor (PAF). However, these factors are not specific to pregnancy and are found in a non-pregnant state as well. More recent work indicates that the embryo's presence, and the products that it secretes, creates a favorable and tolerant environment for a successful pregnancy. Several reports have shown that the embryo-conditioned media has immune-modulatory effects on the maternal organism. The addition of the conditioned culture media, from human and mouse embryos, affects human immune cells activity. This immune modifying activity occurs very early, at the two- cell stage embryo. The activity is dependent on embryo viability, since the media of cultured atretic eggs does not exert such immune-modulatory features. Therefore, shortly after fertilization, the embryo starts actively to emit signals that create maternal recognition of pregnancy which lead to immune tolerance. The cumulus cells, which surround the segregated embryo, may serve as a relay system, since they contain active immune cells that secrete cytokines. Such an intimate contact between putative embryo-derived compounds, and the maternal immune system, would allow for a rapid diffusion of signals from the embryo. This would lead to a local immune response, due to the embryo presence, followed by a systemic maternal immune recognition. Such changes in maternal immunity are shown via a variety of bioassays, including a pre-implantation factor (PIF) assay that measures immune changes in the maternal systemic circulation. This immune change occurs within the first few days after fertilization. Additionally, using IVF cycles, it has been shown that within three days after embryo transfer, PIF activity can be found already in maternal circulation. This indicates that embryo-initiated signaling will rapidly create a systemic immune system tolerance to the embryo. Without wishing to be bound by theory, increased PIF activity may explain why implantation does not necessarily take place in the uterus, but it can occur elsewhere within the organism, and suggest that for embryo transfer to be successful, a similar PIF signal has to exist.

Gonzalez et al., (Am J of Reproductive Immunology, Vol 47, No. 6, 2002) investigates PIF involvement in material immunomodulation, and notes that PIF mimics CD2. WO 03/004601 describes assays for detecting PIF and its use as an early marker of fertilization and embryo viability. Neither Gonzalez et al. nor WO 03/004601 suggest therapeutic use of PIF.

### SUMMARY

Embodiments of the present invention relate to biological effects induced *in vitro* and/or *in vivo* by pre-implantation factor (PIF) peptides as defined in claim 1.
In particular, the present invention relates to use of such PIF peptides to effect changes on the immune system of a patient. More specifically, the addition of the inventive PIF peptides creates specific changes both in cellular immunity as well as in a patient's secreted cytokine profile. Namely, following exposure or stimulation to diverse mitogens, phytohemagglutinin (PHA), CD3 antibody, and the mixed lymphocyte reaction (MLR), PIF peptides of the invention cause a shift towards immune tolerance reducing peripheral mononuclear blood cells (PBMC) proliferation while un-stimulated cells remain unresponsive to the effect of the PIF peptides. Moreover, addition o fnPIF-l₁₅ (SEQ ID NO:1) to stimulated PBMC caused an increase in T_{H}2 (IL10) type cytokine while reducing T_{H}1 (INF-y) type cytokine secretion. Synthetic scrambled PIF peptide (scrPIF-l₁₅), (SEQ ID NO: 5), had no effect on these cells; while addition of synthetic PIF, sPIF-₁₅ (SEQ ID NO: 13) together with the scrPIF-1 (SEQ ID NO: 5) blocked completely the effect of nPIF-l₁₅ (SEQ ID NO:1) on the immune system.

The effects of PIF on the immune response may be measured using flow cytometry and ELISA techniques. Without wishing to be bound by theory, based on the use of fluoroscence labeled PIF peptides, it is believed PIF peptides of the invention bind to specific and likely novel cell surface receptor sites. These PIF receptors appear to be distributed on monocytes, >90% of B cells, and on 4% of un-stimulated and 80% of mitogen-stimulated T cells. Antibodies generated against PIF peptides can be used to develop ELISA and other assays to determine quantitatively or semi-quantitatively levels of PIF in biologic fluids. Any diagnostic procedure whereby compounds of pro-implantation embryo origin that decrease the T_{H}1/T_{H}2 ratio in lymphocytes are used to assess embryo quality in animals and humans. Such assays may be used as part of a diagnostic procedure whereby compounds of pre-implantation embryo origin that decrease the T_{H}1/T_{H}2 ratio in lymphocytes are used to assess the quality of pregnancy in animal and humans or to determine receptivity of the immune system and endometrium prior to pregnancy.

The present invention further relates to the inventive PIF peptides' effect on endometrial cells leading to an increase in a major marker of endometrial receptivity, beta integrin, an adhesion molecule. Specific antibodies against the PIF can be generated by injecting KLH bound peptide and detennining the titer of the antibody produced as the basis for ELISA development. High titer polyclonal antibodies were generated against synthetic nPIF-l₁₅ (SEQ ID NO:1), nPEF-2₁₃ (SEQ ID NO:7) and nPEF-3₁₈ (SEQ ID NO:10) and ELISA assays to measure PIF in biological fluids has been developed, showing differences between pregnant and non-pregnant samples for PIF-1 ELISA (SEQ ID NO:1); determining presence of pregnancy, its viability and outcome in human as well as in other mammals.

PIF peptides of the invention and their receptors could be used beyond pregnancy for diagnostic and therapeutic uses. As a diagnostic, monitoring changes in the PIF receptor that are present on immune cells as well as elsewhere in the body. PIF receptor also could serve as an assay for PIF, where PIF would bind the receptor when present in biologic fluids to determine pregnancy and viability. In addition the inventive PIF peptides, since they modulate the immune system and do not cause basal immune suppression, could be applied to prevent transplant rejection. This is based on the observation that in the MLR system PIF -1 (SEQ ID NO:1) blocked the reaction. This is viewed as an important assay in assessing tolerance development. Further the peptides of the present invention are useful for treatment of autoimmune diseases (including, but not limited to, lupus, arthritis, diabetes) where activated inappropriate immunity plays a key role. The ability to suppress that portion of the immune system that attacks various elements in the body may reduce or prevent these serious debilitating conditions.

### DESCRIPTION OF THE DRAWINGS

In part, other aspects, features, benefits and advantages of the embodiments of the present invention will be apparent with regard to the following description, appended claims and accompanying drawings where:

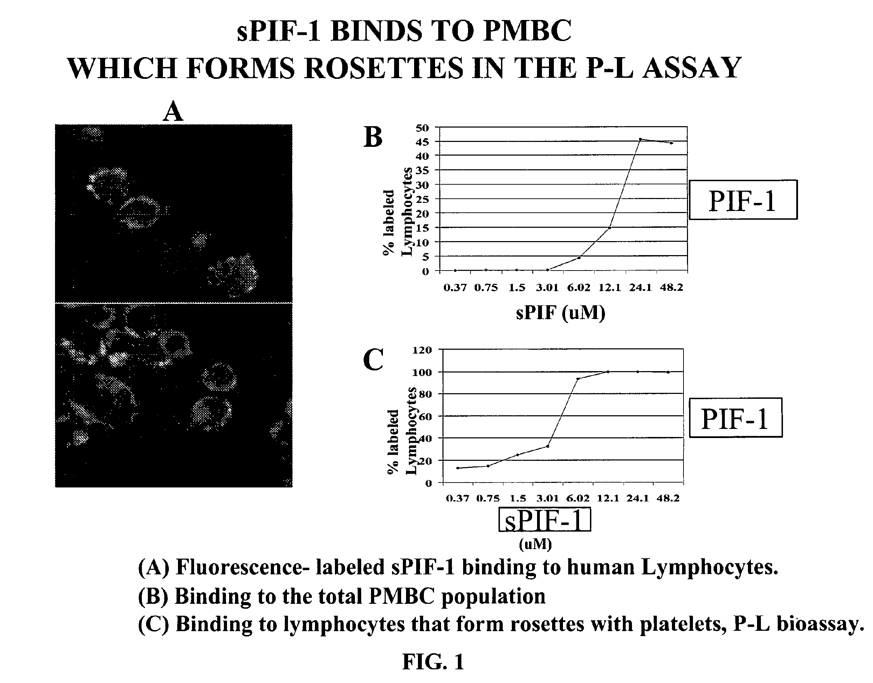

FIG. 1 shows that sPIF-1₍₁₅₎ (SEQ ID NO:13), binds to PBMC, which forms rosettes in their P-L assay. (A) Fluorescence- labeled sPIF-1₍₁₅₎ (SEQ ID NO:13), binding to human Lymphocytes in a dose dependent manner. (B) Binding of FITC sPIF-1₍₁₅₎ (SEQ ID NO:13), to the total PBMC population (C) Binding to lymphocytes that form rosettes with platelets, P-L bioassay, documents presence of nPIF-1₍₁₅₎ (SEQ ID NO:1), receptors on the PBMC surface.

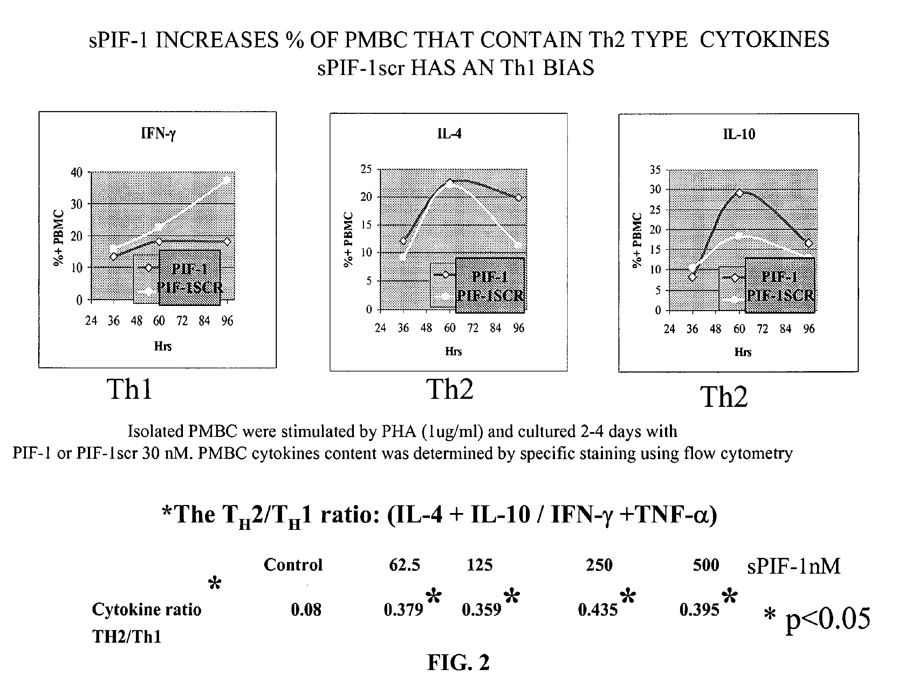

Fig 2. sPIF-1₍₁₅₎ (SEQ ID NO:13) increases the percentage of PBMC that contain T_{H}2 type cytokines (IL10, IL 4 while scrPIF-1₍₁₅₎ (SEQ ID NO: 5) has a T_{H}1 bias (INFgamma). Isolated PBMC were stimulated by PHA (lug/ml) and cultured 2-4 days with sPIF-1₍₁₅₎ (SEQ ID NO:13) or scrPIF-1₍₁₅₎ (SEQ ID NO:5) 30 nM (comparative). PBMC cytokines content was determined by specific staining using flow cytometry.

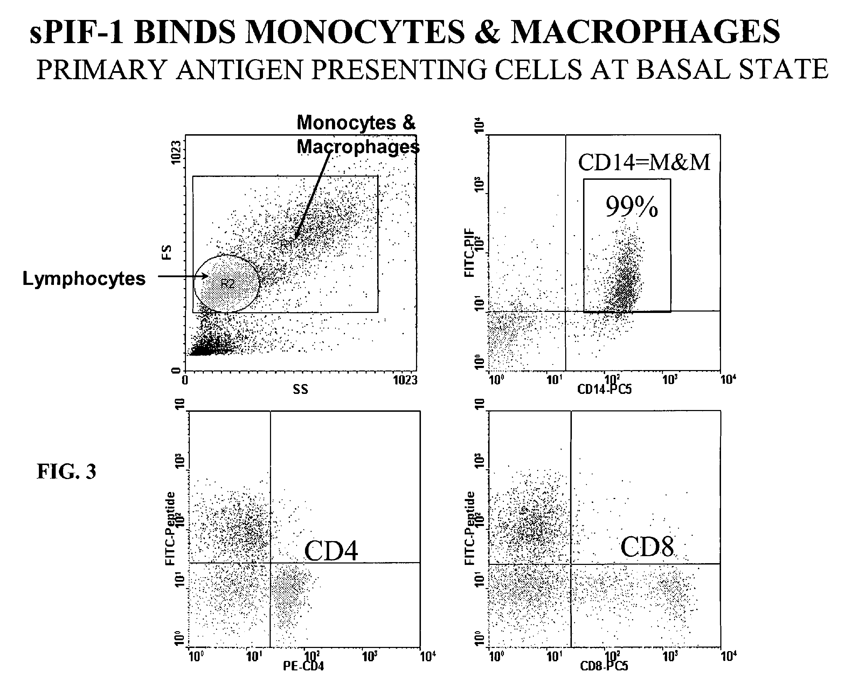

FIG 3. shows preferential binding of FITC sPIF-1 (SEQ ID NO:13) to subpopulation of PBMC. sPIF-1 binds monocytes and macrophages (primary antigen presenting cells) at the basal state. FITC labeled PIF-1 was added to unstimulated PBMC. Binding of the labeled peptide to PBMC subpopulation was determined by using specific CD markers. CD14 + cells represents monocytes.

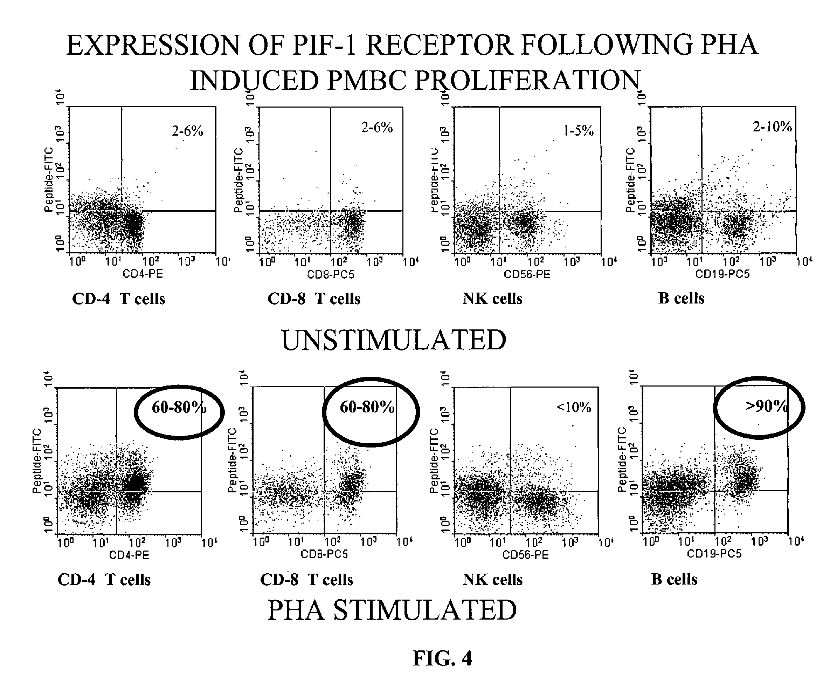

FIG. 4 shows effects of binding by FITC sPIF-1₍₁₅₎ (SEQ ID NO:13) to basal and PHA stimulated PBMC PIF receptors. Expression of PIF-1₍₁₅₎ (SEQ ID NO: 1), receptor following PHA induced PBMC proliferation CD-4 T cells; CD-8 T cells; NK cell and B cells (unstimulated-top); (PHA stimulated- bottom). Flow cytometry analysis of PBMC following exposure to FITC sPIF-1₍₁₅₎ (SEQ ID NO:13), % expression was determined in PBMC subtypes before and after exposure to 1 ug/ml PHA for 24 hours. There was a major increase in PIF-1 (SEQ ID NO:1) receptors expression on T (CD4, CD8 cells, 60-80%) and B cells (>90%) while no changes in the NK cells receptors was noted. This indicates that both T cells (cellular immunity) and B cells (antigen presenting cells) increase PIF-1 receptor expression markedly within 24 hours. While macrophages/monocytes have already a full complement of PIF receptors (first responders). In contrast, minimal receptors expression could be induced by the mitogen on NK (natural killer) cells that are supposed to be protect the body's basal immune response.The delay seen by PIF effects on mitogen induced PBMC cytokines secretion (24h following exposure) may be explained by requirement for receptor induction (takes 24 hours). Therefore only 4 hours exposure had no effect.

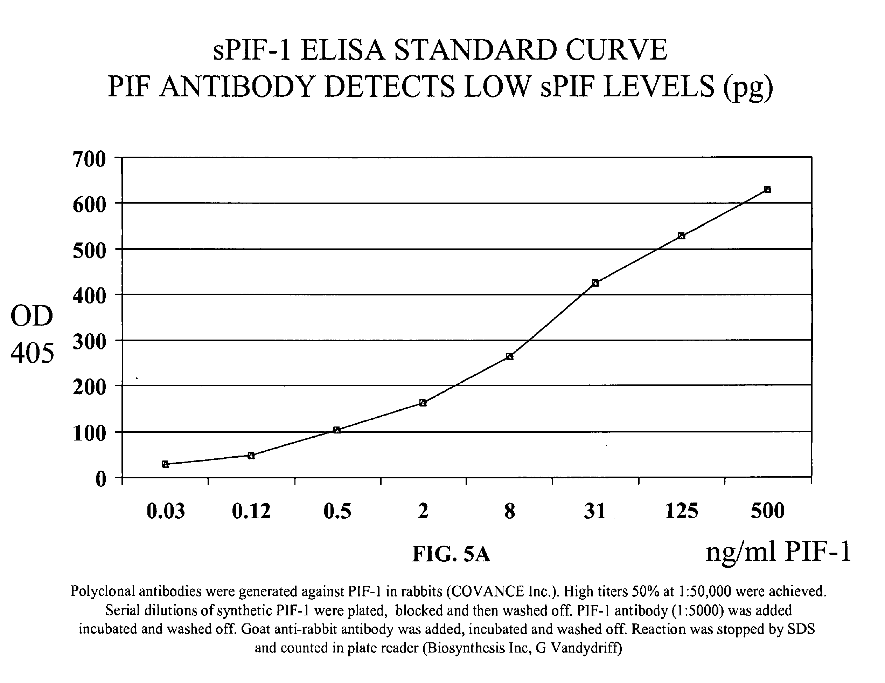

FIG 5A sPIF-1₍₁₅₎ (SEQ ID NO: 13) ELISA standard curve PIF antibody detects low sPIF levels (pg). Polyclonal antibodies AbPIF-1₍₁₅₎ were generated against sPIF-1₍₁₅₎ (SEQ ID NO:13) in rabbits (Covance Inc.). High titers 50% at 1:50,000 were achieved. Serial dilutions of synthetic sPIF-1₍₁₅₎ (SEQ ID NO:13) were plated, blocked and then washed off. PIF-1 antibody (1:5000) was added incubated and washed off. Goat anti-rabbit antibody was added, incubated and washed off. Reaction was stopped by SDS and counted in plate reader (Biosynthesis Inc, G Vandydriff). The antibody affinity was also confirmed by using a competition analysis between biotin labeled and unlabeled sPEF-1₍₁₅₎ (SEQ ID NO: 13) (data not shown). Also, when scrPIF-1 (SEQ ID NO 5) was tested in the assay the antibody did not recognize it attesting to the high specificity of the antibody that was generated. Similar dose dependent results in the ELISA were obtained with affinity purified PIF-2 and PIF-3 antibodies (dilutions of the antibody up to 25,600) with linearity to the 30 pM of the peptide.

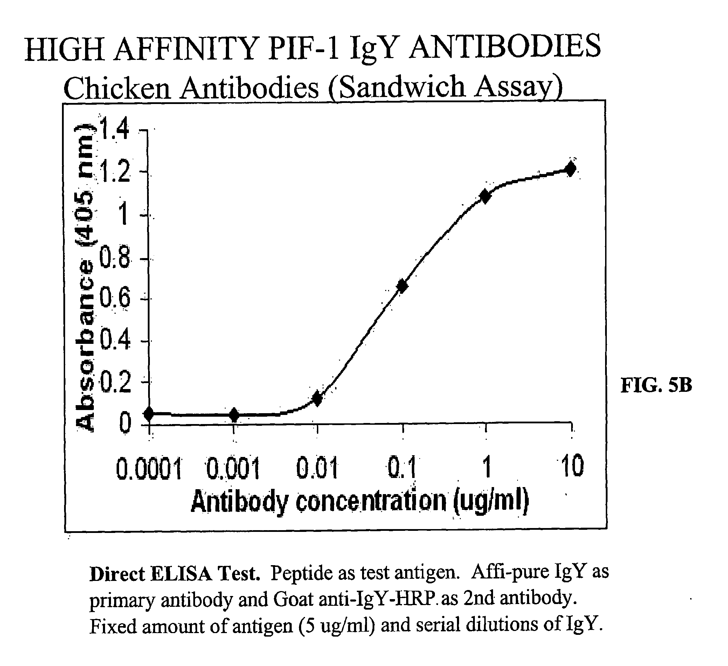

FIG. 5B shows ELISA profile of high affinity PIF-1 IgY antibodies (sandwich assay). Chicken were injected with KLH bound PIF-1 and the eggs were collected and affinity purified on a PIF column.

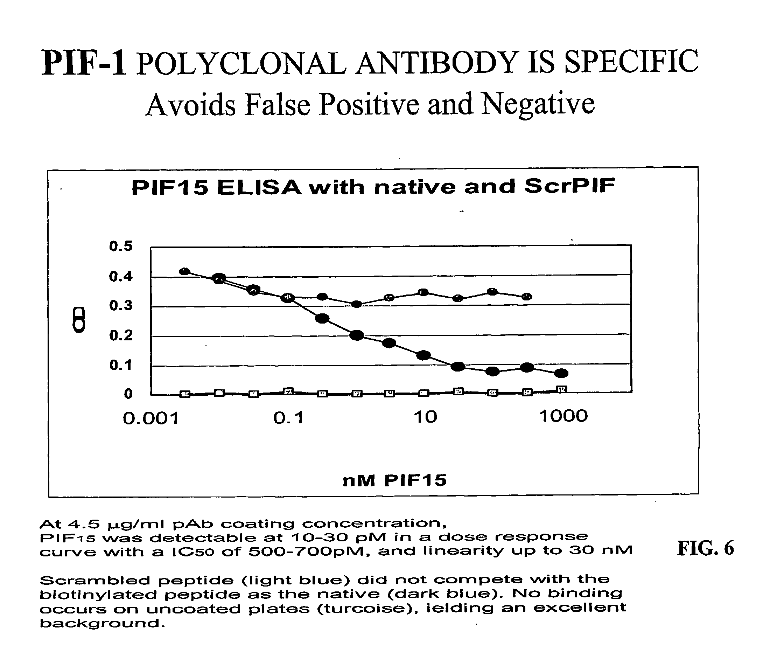

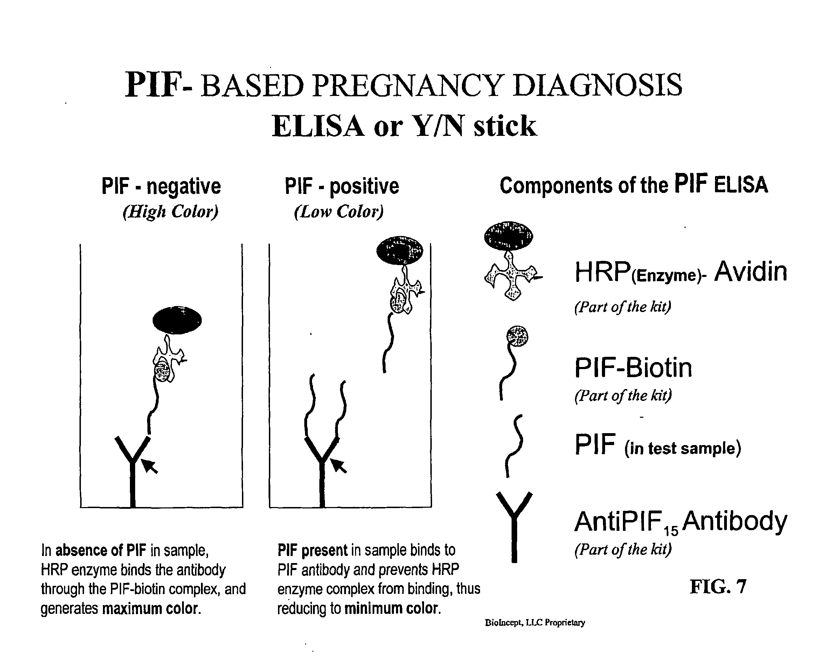

FIG. 6 shows ELISA profile of nPIF-1₍₁₅₎ and scrPIF-1₍₁₅₎ using Biotin labeled versus unlabeled peptide where the antibody captures the peptide in the unknown samples and compares it to standards (see Figure 7).

FIG 7 depicts an example of a PIF-based diagnostic of the present invention. Four clones of monoclonal antibodies to PIF-1₍₁₅₎ were developed as well in mice and ascites fluid was generated with high affinity antibodies as hybridomas with sustained MAb production.

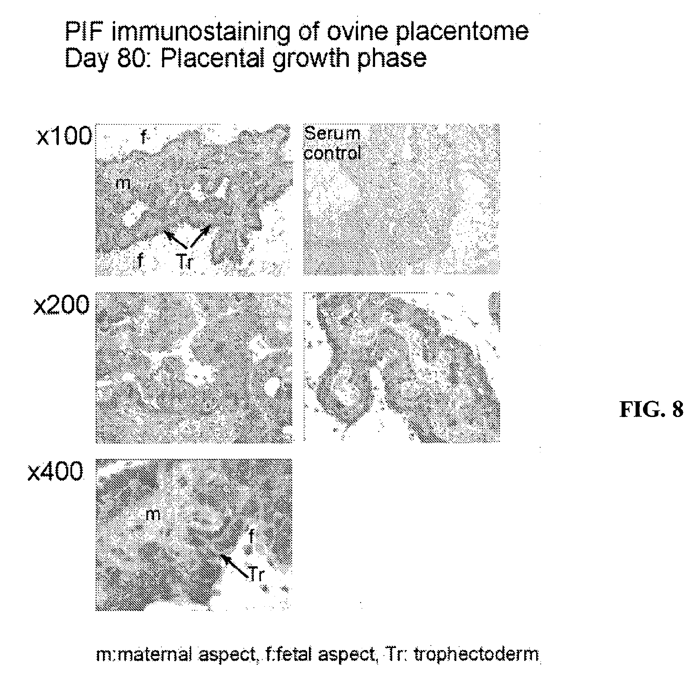

FIG. 8 nPIF-1₍₁₅₎ (SEQ ID NO: 1) is present in the ovine placenta, as demonstrated by immunocytochemistry methods. AbPIF-1₍₁₅₎ was exposed to placental tissue derived from a midtrimester ovine fetus. Compared to the non specific staining by rabbit IgG, the PIF has show intense staining of the fetal portion of the placenta.

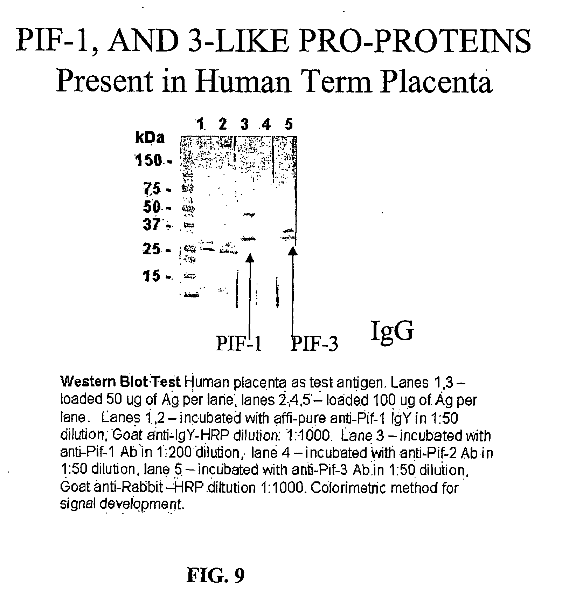

Figure 9. Human term placental extracts were prepared (Dr Jerry Feitelson, GenWay, Inc) and were exposed to PIF antibodies using Western blot analysis, PIF like molecules were stained and the bands obtained were compared to a serial molecular weight standards run in parallel. Results showed that a number of PIF-1 related proteins are present at the 15-40 kDa range PIF-3 had a lower intensity with and was associated with different molecular weight bands. Finally, PIF-2 expression was minimal. This supports the notion that the human placenta may have precursor proteins from which by cleavage PIF peptides are produced. This also supports that view that PIF like molecules are present throughout pregnancy. Finally, it documents that in terms of intensity of expression in human by far PIF-1 is the most relevant at term.

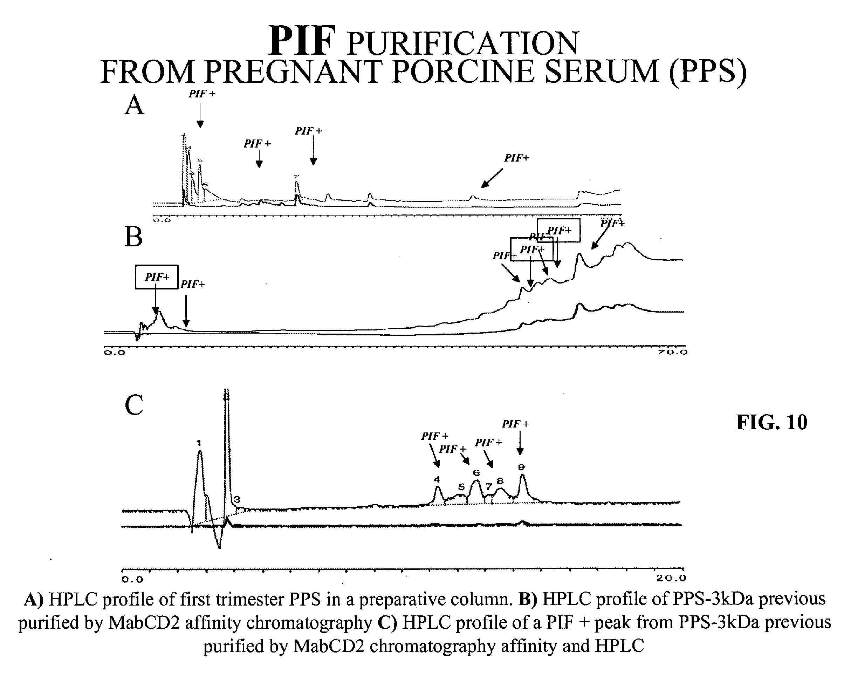

FIG. 10. PIF purification from pregnant porcine serum (PPS). A) HPLC profile of first trimester PPS in a preparative column. B) HPLC profile of PPS-3kDa previously purified by MabCD2 affinity chromatography. C) HPLC profile of a PIF + peak from PPS-3kDa previous purified by MabCD2 chromatography affinity and HPLC.

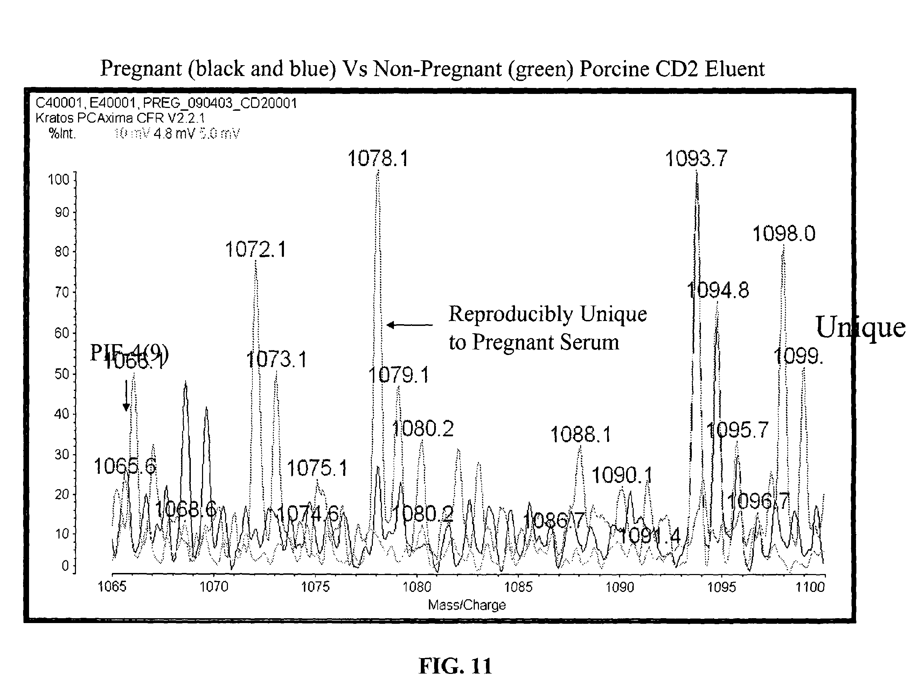

FIG 11. Mass spectrum based identification of PIF peptides in pregnant pig serum as compared to non-pregnant serum following the use of CD2 based affinity column. Mass of the peptides in the non-pregnant v. pregnant samples revealed significant differences at the 900-1100 molecular weight region, where three distinct peptides were identified.

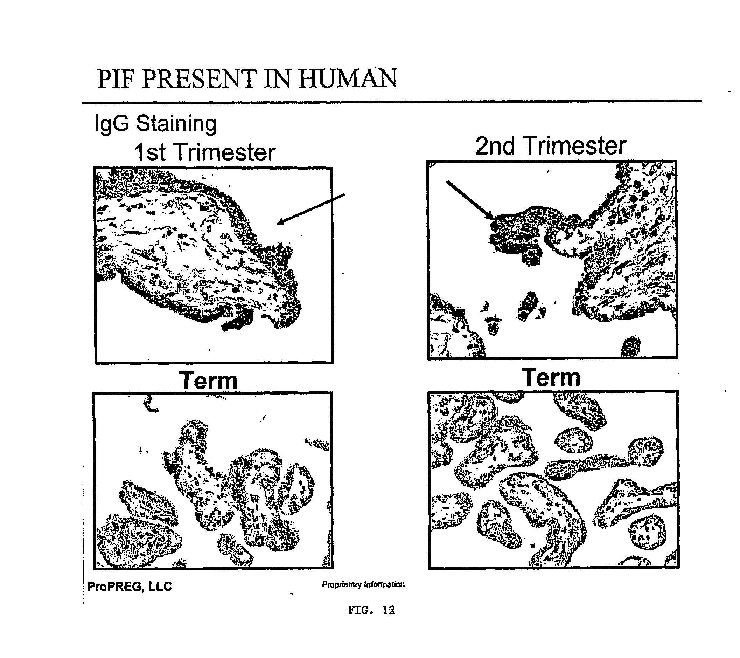

FIG. 12. PIF-1 IgG staining of human placenta during the first trimester, second trimester high in the trophoblastic layer while it declined at term.

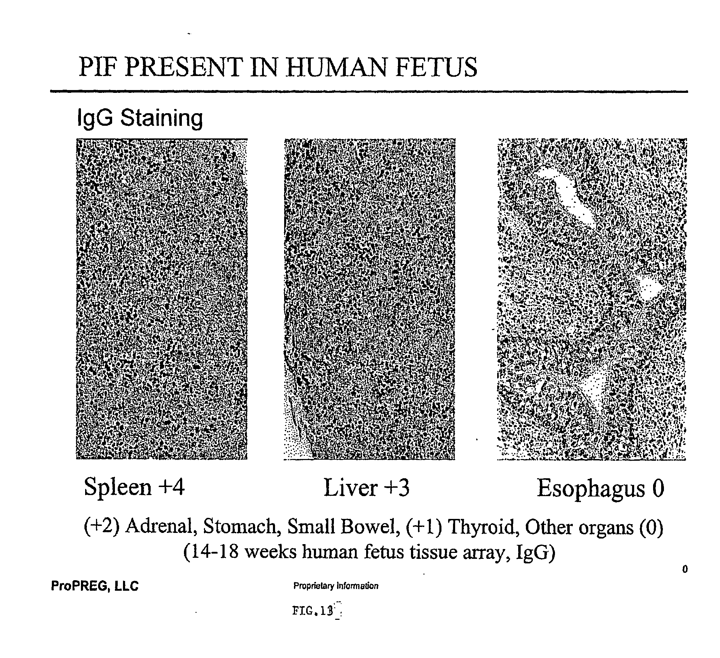

FIG. 13 reflects expression of PIF in various human tissues using IgG in 14-18 weeks human fetus tissue array.

### DETAILED DESCRIPTION

Before the present compositions and methods are described, it is to be understood that this invention is not limited to the particular molecules, compositions, methodologies or protocols described, as these may vary. It is also to be understood that the terminology used in the description is for the purpose of describing the particular, versions or embodiments only, and is not intended to limit the scope of the present invention which will be limited only by the appended claims.

It must also be noted that as used herein and in the appended claims, the singular forms "a", "an", and "the" include plural reference unless the context clearly dictates otherwise. Thus, for example, reference to a "cell" is a reference to one or more cells and equivalents thereof known to those skilled in the art, and so forth. Unless defined otherwise, all technical and scientific terms used herein have the same meanings as commonly understood by one of ordinary skill in the art. Although any methods and materials similar or equivalent to those described herein can be used in the practice or testing of embodiments of the present invention, the preferred methods, devices, and materials are now described.

The PIF assay, as disclosed in U.S. Patent No, 5,646,003 to Barnea et al., entitled "Preimplantation Factor" issued July 9, 1997, and in U.S. Patent 5,981,198 to Barnea et al., entitled "Preimplantation Factor" granted November 9, 1999 may be used to measure the response of the immune system to pregnancy specific preimplantation factors. Studies employing the PIF assay for culture media of human or mouse embryos that were grown, show that PIFs were able to increase the *in-vitro* formation of rosettes between donor lymphocytes and platelets in the presence of monoclonal anti-CD2 (type T11-1). Lymphocyte-platelet rosettes result from the interaction of the T cell surface protein CD2 with its ligand CD58 expressed on the platelet membrane. Anti-CD2, by binding to the CD2 antigen on the T cells, inhibits their interaction with platelets. However, the embryo-derived factor(s), PIFs, present in the culture medium or pregnant peripheral sera appears to counteract this inhibition. The PIF activity was already apparent in the viable two-cell stage embryo. Thus both of those compounds properties are very likely to be similar. This observation strongly suggests that there are several putative compounds that may be very potent, and create an environment that is favorable for pregnancy.

Using this assay, it has been determined that the presence of PIF activity in maternal serum within four days after embryo transfer indicates a >70% chance of successful pregnancy outcome. In contrast, absence of PIF activity indicated that pregnancy would not develop in 97% of cases. PIF is detectable 5-6 days after intrauterine insemination and is absent in non-pregnant serum and in culture media of non viable embryos, present in the sera of various mammals including horse, cow, pig and humans. Without wishing to be bound by theory, the PIF assay results indicate that if the embryo is able to secrete these immunomodulatory PIF compounds, it is capable of implanting and achieving a good pregnancy outcome. The importance of PIF as a marker of a good quality pregnancy is further illustrated by the fact that if a pregnancy ends in miscarriage, the PIF activity progressively declines until it reaches non-detectable levels. In contrast, in the case of a poor quality pregnancy, Human Chronic Gonadotropin (hCG) levels do not change significantly for the next 3 weeks until the miscarriage is clinically evident.

PIF activity is found in several mammalian species, including humans, horse, cow, pig, and mouse and sheep. Human immune cells used for the PIF assay (homologous lymphocytes and platelets) interacted well with the human sera, as well as with sera from different species and embryo culture media. This cross-species interaction indicates that similar compounds are involved in the different species. PIF activity is due to the presence of similar low molecular weight peptides, both in mouse embryo culture media and in pregnant porcine serum. A PIF assay was used as a test to identify and characterize the PIF related compounds within a conditioned mouse embryo culture media. Using a multi-step chromatographic technique, coupled with the PIF bioassay, a group of a putative PIF embryo derived peptides with 9-18 amino acids in length were identified and sequenced. These sequences are disclosed in PCT/US02/20599 to Barnea et al., entitled "New Assays for Preimplantation Factor and Preimplantation Peptides," filed June 28, 2002. Based on the sequences derived, synthetic peptides were generated.

The first natural PIF compound identified, termed nPIF-1₍₁₅₎ (SEQ ID NO:1), is a 15 amino acid peptide. A synthetic version of this peptide, sPIF-1₍₁₅₎ (SEQ ID NO:13), showed activity that was similar to the native peptide, nPIF-1₍₁₅₎ (SEQ ID NO:1). This peptide is homologous to a small region of the Circumsporozoite protein, a malaria parasite. The second PIF peptide, nPIF-2₍₁₃₎ (SEQ ID NO:7), includes 13 amino acids and shares homology with a short portion of a large protein named thyroid and retinoic acid transcription co-repressor, which is identified as a receptor-interacting factor, (SMRT); the synthetic version is sPIF-2 (SEQ ID NO:14). The third distinct peptide, nPIF-3₍₁₈₎ (SEQ ID NO:10), consists of 18 amino acids and matches a small portion of reverse transcriptase; the synthetic version of this peptide sPIF-3₍₁₈₎ is (SEQ ID NO:15). nPIF-4₍₉₎ (SEQ ID NO:12) shares homology with a small portion of reverse transcriptase..

A composition comprising a synthetic PIF peptide and an excipient. In further embodiments, the synthetic PIF peptide corresponds to the amino acid sequence of SEQ ID NO:1, SEQ ID NO:3, SEQ ID NO:4, SEQ ID NO:6, or comprises the amino acid sequence of SEQ ID NO:13 or SEQ ID NO:16, or a combination thereof. In another embodiment, the composition may further include a cell having an expressed PIF receptor bonded to said PIF peptide, for example, embryo cells.

Without wishing to be bound by theory, present evidence suggests that both within the embryos immediately surrounding (found in the fallopian tube, and endometrium *in vivo*), and in the peripheral circulation, similar PIF peptides may be responsible for both the immune effects, and for the creation of a pro-pregnancy environment.

Considerable evidence exists that impaired maternal immune tolerance to the semi-allogeneic conceptus is a cause of implantation failure and pregnancy loss. The distribution of T-helper cell (T_{H}) sub-populations and the resulting local and systemic cytokines balance may play an important role in pregnancy viability. Following antigenic stimulation, T_{H} cells respond by differentiating into one of two cell types: T_{H}1 which produces mainly interleukin 2 (IL-2) and interferon γ (IFN-γ) as well as tumor necrosis factor a (TNF-α); and T_{H}2, which produces IL-4, IL-5 and IL-10. T_{H}-specific cytokines tend to both stimulate proliferation of the T_{H} cell subset from which they are derived and inhibit development of the opposite T_{H} cell subset. T_{H}1 cells are involved in cell-mediated immune reactions while T_{H}2 cells are involved in humoral immunity. A predominance of T_{H}2 cytokines is found in normal pregnancies, and IL-4 and IL-10 released by these cells appear to support pregnancy. In contrast, the T_{H}1 cytokines, IL2, IFN-γ and TNF-α are associated with reproductive failure in both humans and mice. However both types of cytokines are required to maintain pregnancy, since the maternal system must be able to fight aginst infection while tolerating the fetus. It has been postulated that the pre-implantation embryo may play a role in protecting itself from maternal immune rejection by secretion of factors that would promote the shift of T_{H} cells towards the T_{H}2 phenotype. These PIF compounds may be to be used for treatment of inflammatory or other immunological diseases, and preferably the drug or biological is derived from- or its structure is based on the structure of the circumsporosoite protein of malaria.

In one embodiment, the PIF peptides of the invention can be used for both diagnosis and therapy. Non-limiting examples of the effects of such PIF peptides include modulation of the immune system while not causing basal immune suppression, and use of PIF peptide to enhance endometrial receptivity. Such methods of treatment may involve increased expression of endometrial receptivity markers, including, but not limited to beta 3-integrin.

Embodiments of the present invention include those peptides derived from pre-implantation embryós that induces T_{H}2 type cytokines like IL-10 synthesis or secretion from lymphocytes or other white blood cells and pharmacophores that binds specifically to PIF receptors (such but not limited to (A)VRIKPGSANKPSDD or (Q)VRIKPGSANKPSDD) or by substituting with L amino acids or by adding PEG. Preferably such peptides are from pre-implantation embryos and increases T_{H}2/T_{H}1 ratio through increased number of lymphocytes containing the desired cytokines and or by preferential secretion or T_{H}2 over T_{H}1 cytokines into the media. Such pre-implantation embryo-derived peptide may be used to cause a shift from pro-inflammatory to anti-inflammatory activities in lymphocytes.

PIF peptides of the invention may be used to treat a patient by administering to the mother a therapeutic amount of one or more PIFs to create tolerance for the embryo and therefore pregnancy acceptance by the mother. In this embodiment PIF peptides can be used for the treatment of infertility disorders and for the enhancement of pregnancy. Other non-limiting examples where such PIFs may be used include preventing miscarriage and premature labor in mammals such as women, farm, and non-farm animals. The PIF peptides or compositions thereof may be administered using transdermal methods including patch, by injection, or pill, and may include liposome or carbohydrate coated formulations, for example.

In another non- limiting embodiment a PIF peptide of the invention could be added to embryo culture media in order to enhance the ability of the transferred embryos to implant thereby reducing the number of embryos that are needed in order to have a high rate of implantation, and successful pregnancy. PIF could also enhance embryonal viability by acting in an autocrine manner on the embryo itself.

In further embodiments, methods of involving compounds of pre-implantation embryo origin that decrease the T_{H}1/T_{H}2 ratio are used as drugs (biologics) to improve the immune system of the mother to be able to better receive the embryo, as a treatment of infertile women (parenterally or as a co-additive to the embryo cultures in the ET procedure) are provided.

Pre-implantation embryo origin compounds or analogs may be used in procedures that decrease the T_{H}1/T_{H}2 ratio in lymphocytes. In these procedures the PIFs are used as drugs or biologics to treat immunological diseases that benefit from a reduction in the pro-inflammatory activity or enhancement of anti inflammatory activity of the immune system in animals and humans. Alternatively, these compounds can block a decrease in the T_{H}1/T_{H}2 ratio in lymphocytes in which case they are used as drugs or biologics to treat immunological diseases where antibodies are over-produced and inhibition thereof is beneficial in humans and animals. For example, PIF peptides may be administered to non-pregnant patients that have autoimmune diseases like lupus and rheumatoid arthritis where the aim is to reduce the rate of activated immunity while maintaining the basal immunity that is required for defense of the organism. In another example, the compounds of pre-implantation embryo origin are used to decrease the T_{H}1/T_{H}2 ratio in lymphocytes are used to control the function of other proteins that do same effects (*e.g*.. Progesterone Induced Blocking Factor). The administration can be made through non-limiting examples such as a patch, injection, or pill form.
Various aspects of the present invention wil be illustrated with reference to the following non-limiting examples.

**Table 1. PIF Peptides**

| (SEQ ID NO) | Peptide | Amino Acid Sequence |
|---|---|---|
| SEQ ID NO:1 isolated native, matches region of Circumsporozoite protein (Malaria) | nPIF-1₁₅ | MVRXPGSANKPSDD |
| SEQ ID NO:2 * isolated native, matches region of Circumsporozoite protein (Malaria) | nPIF-1₍₁₅₋ₐₗₜₑᵣ₎ | MVRIKYGSYNNKPSD |
| SEQ ID NO:3 isolated native, matches region of Circumsporozoite protein (Malaria) | nPIF-1₍₁₃₎ | MVRIICPGSANKPS |
| SEQ ID NO:4 isolated native, matches region of Circumsporozoite protein (Malaria) | nPEF-1₍₉₎ | MVRIICPGSA |
| SEQ ID NO:5 * synthetic, scrambled amino acid sequence from region of Circumsporozoite protein Malaria | scrPIF-1₁₅ | GRVDPSNKSMPKDIA |
| SEQ ID NO:6 * isolated native, matches region of human retinoid and thyroid hormone receptor-SMRT | nPIF-2₍₁₀₎ | SQAVQEHAST |
| SEQ ID NO:7 * isolated native, matches region of human retinoid and thyroid hormone receptor (SMRT) | nPIF-2₍₁₃₎ | SQAVQEHASTNMG |
| SEQ ID NO:8 * synthetic, scrambled amino acid sequence from region of human retinoid and thyroid hormone receptor SMRT | scrPIF-2₍₁₃₎ | EVAQHSQASTNING |
| SEQ ID NO:9 * | scrPIF-2₍₁₄₎ | GQASSAQMNSTGVH |
| SEQ NO:10 * isolated native, matches region of Rev Trans | nPIF-3₍₁₈₎ | SGIVIYQYMDDRYVGSDL |
| SEQ ID NO: 11 * synthetic, scrambled amino acid sequence from region of Circumsporozoite protein Malaria | Neg control for negPIF-1₍₁₅₎ | GMRELQRSANK |
| SEQ ID NO: 12 * isolated native, matches region of Rev Trans | nPIF-4₍₉₎ | VIIIAQYMD |
| antibody of native isolated nPIF-1₁₅ | AbPIF-1₍₁₅₎ | |
| (SEQ ID NO: 13) synthetic, amino acid sequence from region of Circumsporozoite protein Malaria | sPIF-1₍₁₅₎ | MVRIICPGSANKPSDD |
| (SEQ ID NO: 14) * synthetic, amino acid sequence from of human retinoid and thyroid hormone receptor SMRT | sPIF-₂₍₁₃₎ | SQAVQEHASTNMG |
| (SEQ ID NO: 15) * synthetic, amino acid sequence from region of Circumsporozoite protein Malaria | sPIF-3₍₁₈₎ | SGIVIYQYMDDRYVGSDL |
| (SEQ ID NO: 16) synthetic, amino acid sequence from region of Circumsporozoite protein Malaria | sPIF-1₍₉₎ | MVRIFCPGSA |
| antibody of native isolated nPIF-2₍₁₃₎ | AbPEF-2₍₁₃₎ | |
| antibody of native isolated nPIF-3₍₁₈₎ | AbPIF-3₍₁₈₎ | |
| (SEQ ID NO: 17) * synthetic | sPIF-4₍₉₎ | VIBAQYNm |

| | | |
|---|---|---|
| n=native, s= synthetic, scr =scrambled, same AA, ( ) = number of AA, Ab=antibody * comparative | | |

### EXAMPLE BIOLOGICAL EFFECTS OF PIF PEPTIDES

Preimplantion factor peptides were synthesized by solid-phase peptide synthesis (SPPS, Applied Biosystems Peptide Synthesizer, Model 433) employing Fmoc (9-Buorenylmethoxycarbonyl) chemistry in which the a-amino nitrogen of each amino acid is blocked with Fmoc. Upon completion of the synthesis, final purification is carried out by reversed-phase HPLC and identity is verified by MALDI-TOF mass spectrometry and amino acid analysis. sPIF-1₁₅ (SEQ ID NO:13) and scrPIF-1₁₅(SEQ ID NO:5) scrambled peptide (GRVDPSNKSMPKDIA) and an irrelevant 11 amino acid negative control peptide negPIF-1₍₁₅₎ (SEQ ID NO: 11) (GMRELQRSANK) containing a similar carboxyl-terminal sequence, were synthesized. Peptides were labeled on their N-termini with fluorescein isothyocyanate (FITC) in the solid phase. A spacer group (b-alanine) was inserted between the fluorophor and the peptide. sPIF-1₁₅(SEQ ID NO:13) was also labeled by adding Lysine at the C terminal. In addition, SMRT (SQAVQEHASTNMG) sPIF-2₍₁₃₎ (SEQ ID NO: 14), FITC and Biotin labeled were generated on the N terminal. Scrambled SMRT, scrPIF-2₍₁₄₎ (SEQ ID NO: 9), GQASSAQMNSTGVH; scrPIF-2₍₁₃₎ (SEQ ID NO: 8), EVAQHSQASTMNG; and SGIVIYQYMDDRYVGSDL peptide (reverse transcriptase homologue- RTH) sPIF-3₍₁₈₎ (SEQ ID NO:15) were also generated synthetically.

### PIF-1 PEPTIDES ENHANCE ROSETTES FORMATION.

Figure 1 shows that sPIF-1₍₁₅₎ (SEQ ID NO: 13), binds to PBMC, which forms rosettes in their P-L assay. Figure 1(A) depicts fluorescence- labeled sPIF-1₍₁₅₎ (SEQ ID NO: 13) binding to human lymphocytes in a dose dependent manner. Figure 1(B) shows binding of FITC nPIF-1₍₁₅₎ (SEQ ID NO: 1) to the total PBMC population and Figure 1(C) shows binding to lymphocytes that form rosettes with platelets, P-L bioassay, documents presence of nPIF-1₍₁₅₎ (SEQ ID NO: 1), receptors on the PBMC surface.

Human PBMC were isolated by the Ficoll method. FITC-labeled nPIF-1₁₅ (SEQ ID NO: 1) and (FITC)-labeled scrPIF-1 (SEQ ID NO:5) and match-size irrelevant peptide (FITC)-labeled negative controls negPIF-1₍₁₅₎ (SEQ ID NO:11), were dissolved in PBS at concentrations ranging from 0.75 to 48.19 µM, and were incubated with 500,000 lymphocytes for 1 h at room temperature. The cells were washed 6 times with PBS and resuspended in 500 µl of the same buffer. T-cells and monocytes identification was performed in independent experiments by incubation with anti-CD3 and anti-CD14 antibody-phycoerithryn (PE) labeled. nPIF-1₁₅ (SEQ ID NO: 1) binding to PBMC was determined by flow cytometry. In addition, rosettes formed by T cell-platelets were detected by flow cytometry using anti-CD3 antibody-PE and anti-CD41 a antibody-FITC (all antibodies were from Pharmingen Inc.). These rosettes were not labeled by the control peptides (FITC)-labeled serPIF-1₍₁₅₎ (SEQ ID NO:5) and match-size irrelevant peptide (FITC)-labeled negative controls negPIF-1₍₁₅₎ (SEQ ID NO:11). This indicates that both embryo culture media and serum contain similar peptides and provides evidence for the utility of biologic effects of the peptides and for the diagnostic potential using antibodies against the same.

The biological characteristics of preimplantation factors *in vitro* were determined employing the synthetic versions of both 15-residue sPIF-1₁₅ (SEQ ID NO: 13) and 9-residue sPIF-1₍₉₎ (SEQ ID NO: 16) isoforms, which exhibit similar biological activities *in vitro.* Flow cytometric determination of lymphocyte/platelet rosette formation shows that both _{S}PIF-1₁₅ (SEQ ID NO: 13) and sPIF-1₍₉₎ (SEQ ID NO: 16) isoforms induce a four-fold increase in the number of rosettes in the presence of the anti-CD2 antibody (data not shown), demonstrating that they exhibit the same anti-CD2-blocking effect manifested by the embryo-conditioned culture medium and maternal serum.

In a further example, PIFs effect on PIF bioassay and flow cytometry was observed. Compared to control, the addition of PIF increased platelet/lymphocyte rosette formation as follows:

**Table 2.**

| PIF | Increase in Rosette Formation |
|---|---|
| 1 nM | > 370% |
| 10 nM | > 288% |
| 100 nM | >208% |

Results indicate that synthetic PIF replicates the effects seen by pregnant serum and embryo culture media results.

### PEF-1 IMMUNE EFFECTS

Human PBMC were isolated and cultured for 2-4 days in AIM-V medium containing 0 to 200 nM of sPIF-1₁₅ (SEQ ID NO:13) or scrPIF-1₁₅ (SEQ ID NO:5). The following proliferation-activating agents were used: anti-CD3 antibody (10 µg/ml solution) bound on the plate wells in the presence or absence of IL-2 at 10 µg/ml; phytohemmaglutinin (PHA) at 4 µg/ml. The effects of sPIF-1₁₅ (SEQ ID NO:13) or scrPIF-1₁₅ (SEQ ID NO:5) peptides were also determined by the mixed lymphocyte reaction (MLR) after 3 days in heterologous cultures of PBMC with cells previously treated with mitomycin C (100 µg/ml for 4 h). Cell proliferation was determined by tritiated thymidine incorporation (16 h) of 72-h culture. Cytokine release (IL-4, IL-10, IFN-γ and TNF-α) into culture supernatants was determined at 72 h of culture by ELISA (R&D System).

Overall, sPIF-1₁₅ (SEQ ID NO:13) decreases the proliferation rate of human lymphocytes stimulated with diverse reagents and provokes a shift toward a T_{H}2 cytokine phenotype. sPIF-1₁₅ (SEQ ID NO:13) negatively affects the proliferation of activated lymphocytes. Lower rates of lymphocyte proliferation was found at 250, 62.5 and InM of sPIF-1₁₅ (SEQ ID NO:13) for PHA, anti-CD3 antibody and MLR, respectively. The results were compared with CD3 antibody stimulated lymphocytes without sPIF-1₁₅ (SEQ ID NO:13) or with scrPIF-1₁,₅ (SEQ ID NO:5) used as controls. IL-10 release is significantly increased in the culture supernatants and IFN-γ release is significantly decreased by exposure to sPIF-1₁₅ (SEQ ID NO:13). In contrast, sPIF-1₁₅ (SEQ ID NO:13) does not have a significant effect on IL-4 or TNF-α release, sPIF-1₁₅ (SEQ ID NO:13) increases the TH₂/T_{H}1 cytokine ratio in PBMC more than five-fold, owing mainly to the substantial increase of IL-10 coupled with a decrease in IFN-γ. Number of IL-10 secreting cells also increased (50-60%) starting at day 2 and peaking at days 3-4 and preceding the IFN-γ decreases (30%) by one day, suggesting that a causal relationship is likely in place with respect to the dynamics of these cytokines, as show in Figure 2. In contrast, same concentrations of scrPIF-₁₅ (SEQ ID NO:5) had no effect, as demonstrated by intracellular staining and flow cytometry (Fig. 2).

These results demonstrate that sPIF-1₁₅ (SEQ ID NO: 13) has immunomodulatory effects that may lead to the development of an immune environment that is favorable to or at least tolerant for the presence of the early embryo. The T_{H}2/T_{H}1 cytokine ratio was determined at different concentrations of sPIF-1₁₅ (SEQ ID NO: 13). Similar dose dependent results at the 1-500 nM range were found in PHA and MLR activated lymphocytes by effects of sPEF-1₁₅ (SEQ ID NO: 13) and sPIF-1₍₉₎ (SEQ ID NO: 16). Similar results were obtained with sPIF-2 (SEQ ID NO: 14), compared to scrPIF-2₍₁₃₎ (SEQ ID NO: 8).

The time dependent PIF-1 immunomodulatory effects are demonstrated. A detailed time dependent and multiple controls were used the secretion of several cytokines by PBMC (IL-2, IL-5, IL-8, GM-CSF, IL-4, IL-10, TNF-a, IFN-γβ, IL-1 and IL-6) were measured. Using CD3MAb as mitogen, PIF-1 stimulated mitogen-induced but not basal immune response in a time dependent manner most effects were noted at 24-48 hours. By 4 hours sPIF-1 blocked TNF alpha with recovery by 24 hours. Also INFgamma secretion initiated only after 48 hours. Major increase in a Tₕ2 cytokine (IL10) after 24 hours. was Thus, sPIF-1₁₅ (SEQ ID NO:13) while scrambled peptide, scrPIF-1₁₅ (SEQ ID NO:5) had an early and consitent stimulatory effect only on TNFalpha. Effects on cytokines was already noted at 0.7 nM. Similar effects were found also with other mitogens LPS and PHA when PIF-1 was tested.

### PIF CORRELATES WITH AN IN-VIVO PRO-INFLAMMATORY RESPONSE

PIF activity was measured in normal (n=4) and thrombophilic pregnancies (n=4) using a FC bioassay based upon CD2 binding to Jurkat leukemia cell line. A correlation between PIF activity and IL-1 in normal pregnancies was observed, but not in thrombophilic conditions (p=0.02). No correlation was observed between PIF and TNF α, IL-8 or thrombus precursor protein (TpP) in either normal pregnancies or thrombophilic conditions. Results appear to indicate that the embryo directs maternal immune response, rather than playing a passive role and that PIF may be an early indicator of pregnancy well-being.

### DYNAMICS OF SYNTHETIC PIF-1 INTERACTION WITH RESTING/ACTIVATED PBMC

The expression of PIF binding sites was examined utilizing flow cytometry studies employing FITC labeled sPIF-1₁₅ (SEQ ID NO: 13) and PBMC from normal human donors showed that it binds to a small number of naive T cells. As shown in Figure 3, sPIF-1₁₅ (SEQ ID NO: 13) binds to monocytes and macrophages, the primary antigen presenting cells, at basal state. In contrast, sPIF-1₁₅ (SEQ ID NO: 13) binds all monocytes but not to platelets. Without wishing to be bound by theory, this suggests that monocytes and a sub-set of T cells express prior to pregnancy receptors, and also indicates that although sPIF-1₁₅ (SEQ ID NO: 13) is somehow similar enough to CD2 that it binds to anti-CD2, it does not bind to the platelet marker CD58, as does CD2 itself. PIF-1 sequence has no homology to that of CD2.

Using flow cytometry fluoroscein- labeled sPIF-1₁₅ (SEQ ID NO: 13) binding to immune cells was examined on resting PBMC. Monocytes (CD14+ cells) express binding sites on most cells, while the expression on resting T cells (CD4+ and CD8+), B cells (CD19+) or NK cells (CD56) populations remained very low. As shown in Figure 4, within 24 hrs of activation by PBMC cultured with the T-cell mitogen, phytohemmaglutinin (PHA)), 60-80% of T lymphocytes (T helper cells, T cytotoxic cells), and >90% of B cells became positive for the fluorescent sPIF-1₁₅ (SEQ ID NO: 13) binding, demonstrating that lymphocytes can recognize preimplantation factors and may respond to them but only if activated. NK cells did not appear to bind sPIF-1₁₅ (SEQ ID NO: 13) even after several days in culture with PHA.

### SCRAMBLED PIF-1 SYNTHETIC PEPTIDE ACTS AS ANTAGONIST TO PIF-1.

scrPIF-1₍₁₅₎ (SEQ ID NO:5) has an identical amino acids composition as sPIF-1₍₁₅₎ (SEQ ID NO:13) but the sequence is in random order. When used as a control for sPIF-1₁₅ (SEQ ID NO:13) the FITC scrambled peptide scrPIF-1₍₁₅₎ (SEQ ID NO:5) did bind to PBMC as determined by flow cytometry. Increasing concentrations of scrPIF-1₍₁₅₎ (SEQ ID NO:5) reduced the binding of fluorescent sPIF-1₍₁₅₎ (SEQ ID NO:13) peptide suggesting that scrPIF-1₍₁₅₎ (SEQ ID NO:5) acts on the same receptor as sPIF-1₍₁₅₎ (SEQ ID NO:13). This was further confirmed by the antagonistic effect noted by scrPIF-1₍₁₅₎ (SEQ ID NO:5) effect on PBMC proliferation. PHA or anti-CD3 antibody induced proliferation was dramatically blocked by low concentrations of scrPIF-1₍₁₅₎(SEQ ID NO:5) (125-1000 nM). Addition of the antagonist scrPIF-1₍₁₅₎ (SEQ ID NO:5) (125 nM) blocked also sPIF-1₍₁₅₎ (SEQ ID NO:1) effect on IL10 secretion. scrPIF-1₍₁₅₎ (SEQ ID NO:5) also blocked the effect of sPIF-1₍₁₅₎ (SEQ ID NO:13) induced increase in beta integrin expression by human endometrial cells.

scrPIF-1₁₅ (SEQ ID NO: 5) reverses sPIF-1₁₅ (SEQ ID NO: 14) induced inhibition of PHA triggered PBMC proliferation. Isolated PBMC were stimulated by PHA (4 ug/ml) and cultured 2-4 days with sPIF-1₉ (SEQ ID NO: 16) ( 0-125 nM) +/- scrPIF-1₁₅ (SEQ ID NO: 5) 125/nM. PBMC proliferation was determined by 3H Thymidine incorporation. *= p<0.05.

scrPIF-1₁₅ (SEQ ID NO: 5) also reverses sPIF-1₍₉₎ (SEQ ID NO: 19) induced inhibition of mAbCD3 triggered PBMC proliferation. Isolated PBMC were stimulated by plate bound antiCD3 antibody ( 10 µg/ml) and cultured 2-4 days with sPIF-1₉ (SEQ ID NO: 16) (0-125 nM) +/- scrPIF-1₁₅ (SEQ ID NO: 5) 125/nM. PBMC proliferation was determined by 3H Thymidine incorporation. *= p<0.05.

scrPIF-1₍₁₅₎ (SEQ ID NO: 5) also reverses sPIF-1₍₉₎ (SEQ ID NO: 16) induced IL10 secretion by PBMC following PHA exposure. Isolated PBMC were stimulated by PHA (4 µg/ml) and cultured 2-4 days with sPIF-1₍₉₎ (SEQ ID NO: 16) ( 0-15 nM) +/- scrPIF-1₍₁₅₎ (SEQ ID NO: 5) 125/nM. IL10 secretion was determined by ELISA *= p<0.05.

In scrPIF-1 inhibitory action appears to be exerted on the PIF binding site. Both PIF-1 and scrPIF-1 bind specifically to isolated mouse splenocytes receptors. Based on flow cytometry data it appears that the binding of both peptides was for the same site therefore explaining the clear antagonistic effect of scrPIF-1 on PIF-1 on both cytokine secretion and PBMC proliferation (see below).

Overall, the results from PIF effects on PBMC proliferation, cytokine release and cytokine content of PBMC indicate a major inhibitory effects on cell proliferation and modulation of cytokines secretion and their cellular content. This only noted only under mitogen activated conditions, while PIF alone had no effect. In addition, an increase in some Tₕ1 type of cytokines is also required to protect the maternal organism against infection. The examples of mitogens that were used are highly potent. In contrast, the embryo as it develops on a practical basis until hatching is surrounded by the protective zona pellucida. Therefore the immune reaction towards the embryo by the mother until implantation (where direct contact is established is minimal, and is gradual allowing for development of tolerance in gentle manner. In contrast PIF-1scr has practically no effect except for stimulating TNFalpha, a Tₕ1 type cytokine.

### PIF LIKELY ACTS THROUGH AN INDUCIBLE UNIQUE IMMUNE CELL SURFACE RECEPTOR.

Fluorescent PIF peptides bind to immune cell membranes following activation. In order to better evaluate the receptor site that is an activation marker, flow cytometry (FC) using specific CD marker antibodies was performed. CD69 is expressed during activation of lymphocytes and monocytes, and is a marker of NK cells activation. CD25 is the receptor for IL-2, and a known activation marker as well. The size of these molecules' positive cell population did not correlate with the sPIF-1₁₅ (SEQ ID NO:13) positive populations. This confirmed that PIF receptors in the immune system are unique and selectively expressed on subpopulation of immune cells and they are inducible by mitogenic activation. scrPIF-1₍₁₅₎ (SEQ ID NO:5) inhibitory action appears to be exerted on the PIF binding site.

Splenocytes from virgin female C57BL/6 mice were stained with 300 nM of PIF-1-FITC or scrPIF-1-FITC.

These results show that fluorescent-labeled PIF and scrPIF binding is competed out by unlabeled PIF and scrPIF. scrPIF binding is specific,however, the immune effect is negated (see above).

### PIF-1 ENHANCES ENDOMETRIAL RECEPTIVITY

Human endometrial tissues were digested and stromal and epithelial cells (hEEC) isolated and cultured with a fetal bovine serum (FBS)-enriched medium until confluent layers were obtained. After that the cells were cultured for two more days in a FBS free -medium followed by a further 2-day culture in the same medium containing PIF peptides (1-500 nM). The expression of β-3 integrin by hEEC was qualitatively determined by immunocytochemistry and quantitatively measured by flow cytometry using a specific anti-β-3 integrin monoclonal antibody (SS A6). sPIF-1₁₅ (SEQ ID NO: 13) and 9-residue sPIF-1₍₉₎ (SEQ ID NO: 16) isoforms and sPIF-2₍₁₃₎ (SEQ ID NO: 8) treatment of hEEC exhibits up to a four-fold increase in the expression of β-3-epithelial integrin. These data indicate that preimplantion factor peptides released by the preimplantation embryo up-regulate the expression of an important marker for endometrial receptivity facilitating pregnancy initiation and maintenance.

In addition, to compare the recovery of β-3 integrin expression with PIF effects described after cultured hEEC in a free-fetal bovine serum (FBS) medium some cells were cultured again in FBS-medium. Interestingly, these cells expressed higher levels of β-3-integrin than did cells growing in free FBS- medium but still levels of β-3-integrin expression triggered by PIF effects were higher. In contrast, PIF did not interact with cultures of human endometrial stromal cells. These results could imply that PIF effects on β-3-integrin expression, which indicate an increase of endometrial receptivity, are higher that those triggered by a medium containing progesterone and an undefined variety and concentration of growth factors and hormones. Data using FITC PIF-1 showed that PIF binds to specific sites on epithelial cells.

### PIF IDENTIFIES ABNORMAL IMMUNE RESPONSE OF A PATIENT WITH RECURRENT PREGNANCY LOSS

sPIF-1₁₅ (SEQ ID NO:13) effect was tested on a patient with over 14 miscarriages and no live birth. Her immune cells were examined in the non- pregnant state, by exposure to sPIF-1₁₅ (SEQ ID NO:13) using PBMC preparations. Induction of PIF receptor expression, and binding to lymphocytes were used to characterize the treated cells. Table 3 illustrates the ability of PIF assay to predict premature labor.

**Table 3.**

| Specimen | PHA | (PIF+/CD4+)/CD4+ | (PIF+/CD8+)/CD8+ | (PIF+/CD19+)/CD19+ |
|---|---|---|---|---|
| Control | + | 22.1(0%) | 24.5 | 46.2 |
| Patient | + | 16.2 (-27%) | 16.3 (-33%) | 48.2 |

The patient's PIF receptors appear to be inducible by PHA as are those of the control subject. No differences were observed on B cells, but patient's T cells PIF receptor were ~30% less than the control. Table 4 shows ability of PIF assay to correlate with proinflammatory cytokines in coagulation disorder associated with pregnancy (thrombophylia).

**Table 4. PIF-1 Effects on Cytokine Expression**

| Specimen | PHA | PIF-1₁₅ | TNFα* | IL-10* | T_{H}1/T_{H}2 |
|---|---|---|---|---|---|
| patient | - | - | 7.98 | 7.77 | 1.03 |
| patient | + | - | 14.23 | 24.76 | 0.57 |
| patient | + | + | 18.32 | 24.32 | 0.75(+32%) |
| control | - | - | 4.76 | 4.71 | 0.99 |
| control | + | - | 17.43 | 25.72 | 0.68 |
| control | + | + | 15.46 | 28.18 | 0.55(-19%) |

| | | | | | |
|---|---|---|---|---|---|
| *) All cytokine numbers are % of total PBMC in scatter gate (including CD14) and 4 in table above). | | | | | |

Under sPIF-1₍₁₅₎ SEQ ID NO:13) influence, the T_{H}1/T_{H}2 ratio decreases (as expected) in the control specimen; but the same did not decrease in the patient's PBMC culture media (see bold numbers in table above).

The results obtained with this illustrative clinical case indicate that changes in the immune system of patients with poor obstetric history can be identified prior pregnancy by using PIFs as an embryo surrogate. Such testing can allow for pre-pregnancy identification of patients with poor ability to mount an immune change or tolerance to initiate and maintain pregnancy. Such an insight could help in screening patients at risk of miscarriage and lead to correction of the underlying pathologic condition perhaps by PIF administration. Such testing can be applied during pregnancy as well. Moreover, adding PIF to test the patient's endometrial cells properties (beta integrin or such), following collection by biopsy may help to determine whether the mother is able to respond to the presence of the embryo by increased receptivity.

### GENERATE POLYCLONAL ANTIBODIES TO THE SYNTHETIC PIF-1, PIF -2 AND PIF-3 PEPTIDES

To generate specific antibodies against sPIF-1₁₅ (SEQ ID NO:13) conjugation to carrier, Keyhole Limpet hemocyanin (KLH) was carried out. sPIF-1₁₅ (SEQ ID NO:13) was conjugated to KLH either on the carboxy or amine terminus of the molecule to cover potential differences in immunogenicity related to peptide presentation. The two peptide-carrier conjugates generated were injected into two rabbits. Within a 5-week immunization protocol all 4 rabbits responded by generating a high titer serum, with a titer of 1:50,000-1:150,000. The titer strength appeared to increase with the second bleeding. These rabbits may serve as a longterm reservoir of serum for antibody generation AbPIF-1₍₁₅₎. The rabbits may continue to be injected with immunogens on a monthly basis, collecting sera periodically and testing for titer and affinity. Antibodies to other PIFs, including AbPIF-2₍₁₃₎ and AbPIF-3₍₁₈₎, were generated with the same method using KLH bound peptide in the amine terminal. Rabbits bled 8 weeks after immunization yielded 1:25,000 titers for both peptides with detection of the PIF peptides to the nanomolar region. These antibodies were affinity purified using PIF-1, PIF-2 and PIF-3 bound affinity columns. The purified antibodies were conjugated each to a separate affinity column and they will serve for isolation of PIF peptides from various biological fluids.

Monoclonal antibodies to PIF-1 were developed as well. A hybridoma cell that produces a monoclonal antibody specific for a PIF-polypeptide, and culturing the cell under conditions that permit production of the monoclonal antibody.

Such PIF antibodies may be used in assay as well as in therapeutic treatment (vaccination) of patients. For example, PIF peptide conjugates may be used as antigen (vaccine) to fight malaria. PIF itself, being a minimal unit might behave as a better antigen than the when its sequence is embedded in the intact, full length circumsporozoite protein in the malaria outer cell membrane. In another example, PIF antagonist (a peptide or other chemical shown to bind to PIF receptors, and block PIF function) or any procedure whereby such compound is used as drug, may be useful to treat malaria or block malaria propagation in the human body (by blocking the sites through/by which the parasite controls and paralyses the immune system and allows it to proliferate). Similarly, any humanized or horse antibodies to PIF or a procedure whereby these are used as agents may be used for passive immunization for malaria. (assuming such antibodies must recognize the circumsporozoite protein on the malaria parasite).

In one example, polyclonal antibodies AbPIP-1₍₁₅₎ were generated against sPIF-1₍₁₅₎ (SEQ ID NO: 13) in rabbits (Covance Inc.). High titers 50% at 1:50,000 were achieved. Serial dilutions of synthetic sPIF-1₍₁₅₎ (SEQ ID NO: 13) were plated, blocked and then washed off. PIF-1 antibody (1:5000) was added incubated and washed off. Goat anti-rabbit antibody was added, incubated and washed off. Reaction was stopped by SDS and counted in plate reader (Biosynthesis Inc, G Vandydriff). As shown in the ELISA standard curve of Figure 4, PIF antibody detects low sPIF levels (pg). The antibody affinity was also confirmed by using a competition analysis between biotin labeled and unlabeled nPIF-1₍₁₅₎ (SEQ ID NO: 1) (data not shown). Also when scrPIF-1 (SEQ ID NO 5) was tested in the assay the antibody did not recognize it attesting to the high specificity of the antibody that was generated.

Figure 5A demonstrates the affinity of PIF-1 IgY antibodies. Peptide as test antigen. Affi-pure IgY as the primary antibody and goat anti-Ig-Y as the secondary antibody. A fixed amount of antigen (5 µg/ml) and serial dilution of IgY.

Figure 5B demonstrates the specificity of PIF-1 polyclonal antibody. At 4.5 µg/ml pAb coating concentration, PIF-1₁₅ was detectable at 10-30 pM in a dose response curve with an IC₅₀ of 500-700 pm, and linearly up to 30 nM. scrPIF-1 did not compete with biotinylated peptide, as the native. No binding appears to occur on uncoated plates, yielding a good background. Results demonstrate that PIF-1 polyclonal antibody appears to avoid false positives and negatives.

Such antibodies could be useful for a PIF-based pregnancy diagnosis utlilizing an ELISA or yes/no stick in the form of a kit. The components of the PIF ELISA kit may include, for example, HRP-Avidin, PIF-Biotin and anti-PIF-1₁₅ antibody. In the absence of PIF in the test sample, HRP enzyme would bind to the antibody through the PIF-biotin complex, generating a maximum color. In the presence of PIF in the test sample, PIF binds to the PIF antibody and prevents the HRP enzyme complex from binding, generating a minimum color.

### ISOLATION AND IDENTIFICATION OF PIF LIKE PROTEINS IN HUMAN PLACENTA AND OTHER FETAL TISSUES

Using affinity purified PIF IgG 1, 2 and 3 and Igy PIF-1, PIFs were identified in human term placenta using Western blot. Human placenta was the test antigen. Lanes 1 and 3 were loaded with 50 µg of antigen per lane and lanes 2, 4 and 5 were loaded with 100 µg of antigen per lane. Lanes 1 and 2 were incuabeted with affi-pure and-PIF-1 igY in a 1:50 dilution, goat anti-IgY-HRP in a 1:1000 dilution. Lane 3 was incubated with anti-PIF-1 antibody in 1:200 dilution. Lane 4 was incubated with anti-PIF-2 antibody in 1:50 dilution. Lane 5 was incubated with anti-PIF-3 antibody in 1:50 dilution and goat/anti-rabbit-HRP in a dilution of 1:1000. Results of western blot are shown in Figure 9.

Expression of PIF-1 in human pregnancy tissues was examined using affinity purified IgG using immunohistochemistry methods. Intense trophoblastic expression was found in first and second trimester placenta while expression was low at term. With respect to the 14-18 weeks fetus using a tissue array (60 samples, covering practically all organs). The highest expression was in the spleen and liver, with lesser in the adrenal, stomach and small bowel with no detectable expression in the esophagus and several other organs. The presence of PIF was also measured in the adrenal tissue, stomach, small bowel, thyroid and other organs (not shown). Non relevant IgG was used as controls.

Overall this indicates that PIF-1 is expressed in the human placenta and fetus across gestation where it declines at term to facilitate the process of delivery by lowering maternal tolerance for the fetus. With respect for the fetus highest expression are found in hemopoietic organs where immune reaction is expected to be the highest.

In a placental sample derived from a patient with premature labor at mid-trimester, PIF expression decreases significantly as examined with the affinity purified PIF-1 IgG antibody. The decline correlated with that of IL10, a major Th2 cytokine. When placental explants are placed in culture and interferon gamma is added, then PIF is reexpressed back to the intensity that was observed in mid trimester. This suggest a reciprocal relationship between cytokines and PIF.

In another example, PIF-1 associated proteins were identified in human placental tissue. Term human placental homogenates were passed through an affinity column of PIF-1 antibody. The mass spectrometry profile following elution by PIF-1 antibody affinity column. Various PIF-1 associated proteins were identified and sequenced following affinity chromatography, including (NM_000039) apolipoprotein A-I precursor [Homo sapiens], electron-transferring-flavoprotein dehydrogenase, (BC017165) similar to triosephosphate isomerase 1 [Homo sapiens], (NM_052925) leukocyte receptor cluster (LRC) member [Homo sapiens], (NM_018141) mitochondrial ribosomal protein S10; mitochondrial 28S ribosomal protein S10 [Homo sapiens], (NM_00518) beta globin [Homo sapiens], (BC012292) heat shock 27kDa protein 1, stress responsive, estrogen regulated [Homo sapiens] P04792.40, Estradiol beta 1 dehydrogenase 1 P14061.13, Fetal Beta MHC binding factor Q 14297.01, microtubule-associated protein 1A (proliferation-related protein p80 P78559.40). Some of those proteins were not previously described in the placenta. The proteins sequenced appear to show roles in immune function, cytoskeleton, enzyme function, and protein synthesis and cell proliferation. None of the sequenced proteins have sequence homology with PIF-1, therefore it likely reflects, in some cases, that PIF is attached to these proteins reflecting a protein-protein interaction related to the peptides function.

### DEMONSTRATION THAT PIF IS PRESENT IN THE PLACENTA OF SHEEP

Placental tissues were collected from a mid-gestation sheep fetus. The placenta was embedded in paraffin and slides were prepared. Representative slides exposed to the 1/100 dilution of rabbit AbPIF-1₍₁₅₎ antibody. Compared with the non immunized serum, AbPIF-1₍₁₅₎ antibody intensely stained the placenta, as shown in Figure 8. The DAKO Chemmate system on the autostainer with DAB as the substrate was used. Moreover, the binding was highly specific since no adjacent maternal tissues appeared to be stained by the antibody (Fig. 8). As such, AbPIF-1₍₁₅₎ antibody as well as AbPIF-2₍₁₃₎ and AbPIF-3₍₁₈₎ could be useful in identifying presence of PIF in pregnant tissues. Gestational changes: Visual analysis was carried out on day 50 (n = 4), day 80 (n = 7), day 100 (n = 7), day 128 (n = 4) and day 135 (n = 8) 9Term =145 days). Based on visual assessment of percent staining, placental levels were highest at day 50 and then declined to day 80 after which they remained constant. This pattern reflected the observations that were made with the human placenta.

In terms of localization, PIF-1 is localized to the ovine maternal-fetal interface which is comprised of fetal trophoblast and maternal epithelium. Interestingly, PIF-1 is localized to the binucleate trophoblast cells. These are non-proliferative migratory cells which fuse with the maternal epithelium to form a hybrid maternal-fetal syncytium. On many of the slides there appears to be epithelial staining. Some of this may come from the fetal contribution to this layer. Later in gestation, the staining becomes more restricted to the binucleate trophoblastic cell population.

PIF-1 expression decreases at term of growth restricted fetuses. A comparison of the placental growth restricted group (n =14) to the controls (n = 14) at day 80 (placental growth period) gave no significant differences. In late gestation, there is evidence of a decrease in PIF1 in the growth restricted group compared with controls.

### ISOLATION OF PIF PEPTIDES FROM SERUM OF PREGNANT MAMMALS

Blood was collected from known pregnant sow. Serum was separated by centrifugation and the serum was stored at 20C until use. The collected serum was filtered through a <3kDa Amicon membrane. The filtrate was further subjected to a CD2 affinity column, as shown in Figure 10. The collected samples were prepared and mass spectroscopy used to determine the molecular weight of samples by MALDI-TOF workstation. The molecular weight of samples was compared with non-pregnant porcine samples prepared in parallel. A number of peptides unique to pregnancy were isolated molecular weight range of about 1030-1100 daltons as shown in Figure 11.

In another example, PIF-1 ELISA was used to detect pregnancy in pigs and cows. Using the PIF-1 ELISA competitive Biotin assay, known pregnant and non-pregnant samples were tested. Heat inactivated serum samples were diluted 1/30 and 1/100 and a low dilution difference between pregnant and non-pregnant samples were found for sows and a cow. The estimated PIF-1 in the pregnant sera are 10-30 nM. The heat inactivation helped to reduce the background of the assay generated by some high molecular weight proteins that were recognized by the PIF-1 antibody seen be Western blot (data not shown).

In a further example, serum samples were obtained at weekly intervals from twenty (20) women in a cross-sectional study (eight (8) in the first trimester, seven (7) in the second trimester, and five (5) in the third trimester) and two (2) women from 5-40 weeks gestation. All women had measurable PIF activity present in serum samples using bioassay. No PIF activity was detected in umbilical cord serum using bioassay. Results indicated that PIF is present throughout various stages of pregnancy.

### GENOME ANALYSIS

Ten genes isolated from human placenta were analyzed. Seven of the 10 genes were annotated for both human (Hs) and mouse (Mm) genomes. When these were researched on the mpuse microarray dataset for preimplantation development, using either Mm or Hs unigene identifiers returned the same result. The arrays represent four replicated samples averaged from 2AFFY chip platforms. Three of the genes (HSP1, BHSDH, MAP1A) did not change; however, four genes (mRP-S10, ApoA-1, FDH, TPI) were all upregulated in the blastocyst and there may be a weak tendency for this increase as early as the 8-cell stage.

### PIF-1 AND SCR PIF-1 CONTRACEPTIVE EFFECTS IN MICE

Preliminary *in vivo* assessment of PIF-1 antagonist by using PIF1scr/amide. With the view that PIF-1scr is a peptide, which, in general has a short half-life, estimated to be 30 minutes, a PIF-1 scr was generated that had an additional amide group at the C terminus. This was an attempt to make the molecule more stable and have a longer half-life. Following the protocol below: (Dr Hoberman, Argus, Inc) PIF-1scr/amide was introduced two days after estrus to mice through an osmotic mini-pump (Alzet Model 2001; 1 ul/hr for 7 days) containing either 0.9% saline or 150 ug or 800 ug PIF-1scr/amide/day release in saline. The pumps were inserted subcutaneously under ketamine/xylazine anesthesia. Female mice were placed with male mice on the 3rd day afternoon of the expected estrus. Four different groups 5 each were studied, low and high dose PIF-1scr/amide, saline control, and one group with PIF-1. Mating was confirmed by the presence of sperm in the vagina or a copulatory plug the next morning. Pregnancy (or lack thereof) was determined by sacrificing on day 10 after breeding and the uterine horns were examined for implantation sites by using Chicago Blue solution. Results showed a trend towards lower number of implantation sites at the lower PIF-1scr/amide dose v. control group. This however did not reach statistical significance. No differences between high dose PIF-1scr/amide and controls were noted.

PIF-1 has no toxic effects following administration in early pregnancy to pregnant mice. This is evidenced by no significant effect on mouse fetuses number as well there was no effect on maternal body weights were noted among all the tested groups.

### SCRAMBLED PIF-1 BY INTRAVAGINAL ADMIMSTRATION MAY HAVE A NON-TOXIC CONTRACEPTIVE EFFECT

Female mice were mated with mice from the same strain. Subsequently, on day 0 (presumed day of mating) PIF-1 scr in saline solution was administered intravaginally twice daily for seven days (Dr Alan Hoberman, Argus, Inc). The dose was 800 µg/day, 150 µg/ml or saline vehicle only (5 animals in each group). Subsequently, mice were sacrificed at day 12 of presumed gestation and Caesarean-sectioned. Corpora lutea, implantation sites and live and dead embryos were recorded. A total of 2 animals in each of the treatment groups and one mouse in the control group failed to conceive. 4/10 of treated while 1/5 control animals conceived, the effect actually being all or none. No toxic or teratogenic effects were noted following the PIF administration since the number of implantation sites and viable embryos were unaffected in those mice that conceived

### PRELIMINARY STUDY: PIF-1 ANTIBODY AND SCRAMBLED PIF-1 INTRAVENOUS ADMINISTRATION IS NON TOXIC

The contraceptive effect of either 150 ug of PIF-1scr in DMSO or 10 µg affinity purified PIF-1 (10 animals per group) or 20% DMSO solution (used as controls) was tested using single daily intravenous injections (Dr Alan Hoberman, Argus, Inc). Female mice were placed with male mice on the 3rd day afternoon of the expected estrus. Mating was confirmed by the presence of sperm in the vagina or a copulatory plug the next morning. Subsequently, were injected for 5 days one injection/day. Mice were sacrificed at day 12 of presumed gestation and Caesarean-sectioned. Corpora lutea, implantation sites and number of live and dead embryos were recorded. 2/10 mice in the PIF-1a and PTF-1 antibody group did not get pregnant, while in the control group all mice were pregnant. The effect was all or none since no toxic or teratogenic effects were noted in the mice that conceived.

Although the present invention has been described in considerable detail with reference to certain preferred embodiments thereof, other versions are possible.

### REFERENCES

1. S.R. Choudhury, L.A. Knapp, Hum. Reprod. Update 7,113 (2001).
2. A.K. Abbas, K.M. Murphy, A. Sher, Nature 383, 787 (1996).
3. R. Raghupathy, Immunol. Today 18,478 (1997).
4. T.G. Wegmann, H. Lin, L. Guilbert, T.R Mosman, Immunol. Today 14,353 (1993).
5. E.R. Barnea, K.I. Lahijani, R. Roussev, J.D. Barnea, C.B. Coulam, Am. J. Reprod. Immunol. 32, 133 (1994)
6. R.G. Roussev et al., Mol. Human Reprod. 2, 883 (1996)
7. R.G. Roussev, E.R. Barnea, E.J. Thomason, C.B. Coulam, Am. J. Reprod. Immunol. 33, 68 (1995).
8. C.B Coulam, R.G. Roussev, E.J. Thomasson, E.R. Barnea, Am. J. Reprod. Immunol. 34, 88(1995).
9. E.R Barnea et al., Am. J. Reprod. Immunol. 42, 95 (1999).
10. E.R. Barnea, C.B. Coulam, US Patent 5981198 (1999).
11. A.C. Cavanagh, H. Morton, Eur. J. Biochem. 222, 551 (1994).
12. E. Critser, J. English, in Immunological Obstetrics, C.B. Coulam, W.P. Faulk, J.A. McIntyre, Eds. (W.W. Norton, New York, 1992), pp. 202-215.
13. S. Heyner, Early Preg.3, 153 (1997).
14. Circumsporozoite protein-malaria parasite (Plasmodium falciparum) (isolate NF54) (Protein accession number S05428)
15. G. Taubes, Science 290, 435 (2000).
16. SMARC32 a putative sperm transmembrane protein related to circumsporozoite protein (Protein accession number AAG31422).
17. D.L. Bodian, E.Y. Jones, K. Harlos, D.I. Stuart, S.J. Davis, Structure 2, 755 (1994).
18. M.P. Piccinni et al., Eur. J. Immunol., 8, 2431 (2001)
19. S.N. Wickramasinghe, S.H. Abdalla, Baillieres Best. Pract. Res. Clin. Haematol. 13, 277 (2000).
20. S. Romagnani, Annu. Rev. Immunol. 12, 227 (1994).
21. G. Chaouat et al., Immunol. 154, 4261 (1995).
22. H. Hong-Nerng et al., Fertil. Steril. 76, 797 (2001).
23. M.Y. Wu et al., Am. J. Reprod. Immunol. 46,386 (2001).

## Claims

1. One or more PIF peptides selected from the group consisting of SEQ ID NO: 1, SEQ ID NO: 3, SEQ ID NO: 4, SEQ ID NO: 13, SEQ ID NO: 16 and combinations thereof for use in the treatment of infertility, prevention of miscarriage or premature labour in a female patient.

2. The peptide(s) of claim 1, wherein the amount of one or more PIF peptides increases the amount of TH2 cytokines in said patient.

3. The peptide(s) of claim 1, wherein the amount of one or more PIF peptides administered to said patient creates a tolerance in said patient for embryo implantation.

4. The peptide(s) of claim 3, wherein the amount of one or more PIF peptides administered to said patient enhances endometrial receptivity.

5. The peptide(s) of claim 3, wherein enhancement of endometrial receptivity is measured by an increase in beta-3-integrin expression.

6. The peptides(s) of claim 1, wherein the PIF peptide is SEQ ID NO: 1.

7. The peptides(s) of claim 1, wherein the PIF peptide is SEQ ID No: 3.

8. The peptides(s) of claim 1, wherein the PIF peptide is SEQ ID NO: 4.

9. The peptides(s) of claim 1, wherein the PIF peptide is SEQ ID NO: 13.

10. The peptides(s) of claim 1, wherein the PIF peptide is SEQ ID NO: 16.

## Patentansprüche

1. Ein oder mehrere PIF-Peptide, die aus der Gruppe, bestehend aus SEQ ID NO: 1, SEQ ID NO: 3, SEQ ID NO: 4, SEQ ID NO: 13, SEQ ID NO: 16 und Kombinationen davon, ausgewählt sind, zur Verwendung bei der Behandlung von Unfruchtbarkeit, Verhinderung von Spontanabort oder vorzeitigen Wehen bei einer Patientin.

2. Peptid(e) gemäß Anspruch 1, wobei die Menge des einen oder der mehreren PIF-Peptide die Menge an TH2-Zytokinen der Patientin erhöht.

3. Peptid(e) gemäß Anspruch 1, wobei die Menge des einen oder der mehreren PIF-Peptide, die der Patientin verabreicht wird, in der Patientin eine Toleranz gegenüber Embryoimplantation erzeugt.

4. Peptid(e) gemäß Anspruch 3, wobei die Menge des einen oder der mehreren PIF-Peptide, die der Patientin verabreicht wird, die Empfängnisbereitschaft des Endometriums steigert.

5. Peptid(e) gemäß Anspruch 3, wobei die Steigerung der Empfängnisbereitschaft des Endometriums über eine Zunahme der Expression von Beta-3-Integrin gemessen wird.

6. Peptid(e) gemäß Anspruch 1, wobei das PIF-Peptid SEQ ID NO: 1 ist.

7. Peptid(e) gemäß Anspruch 1, wobei das PIF-Peptid SEQ ID NO: 3 ist.

8. Peptid(e) gemäß Anspruch 1, wobei das PIF-Peptid SEQ ID NO: 4 ist.

9. Peptid(e) gemäß Anspruch 1, wobei das PIF-Peptid SEQ ID NO: 13 ist.

10. Peptid(e) gemäß Anspruch 1, wobei das PIF-Peptid SEQ ID NO: 16 ist.

## Revendications

1. Un ou plusieurs peptides PIF sélectionnés dans le groupe consistant en SEQ ID NO : 1, SEQ ID NO:3, SEQ ID NO:4, SEQ ID NO : 13, SEQ ID NO : 16 et des combinaisons de ceux-ci pour utilisation dans le traitement de l'infertilité, la prévention de fausse-couche ou d'accouchement prématuré chez un patient de sexe féminin.

2. Le ou les peptide(s) de la revendication 1, où la quantité d'un ou de plusieurs peptides PIF augmente la quantité de cytokines TH2 chez ladite patiente.

3. Le ou les peptide(s) de la revendication 1, où la quantité d'un ou de plusieurs peptides PIF administrés à ladite patiente crée une tolérance chez ladite patiente pour l'implantation d'embryon.

4. Le ou les peptide(s) de la revendication 3, où la quantité d'un ou de plusieurs peptides PIF administrés à ladite patiente renforce la réceptivité endométriale.

5. Le ou les peptide(s) de la revendication 3, où le renforcement de la réceptivité endométriale se mesure par une augmentation de l'expression de bêta-3-intégrine.

6. Le ou les peptide(s) de la revendication 1, où le peptide PIF est SEQ ID NO : 1.

7. Le ou les peptide(s) de la revendication 1, où le peptide PIF est SEQ ID NO : 3.

8. Le ou les peptide(s) de la revendication 1, où le peptide PIF est SEQ ID NO : 4.

9. Le ou les peptide(s) de la revendication 1, où le peptide PIF est SEQ ID NO:13.

10. Le ou les peptide(s) de la revendication 1, où le peptide PIF est SEQ ID NO:16.


### SEQUENCE LISTING

<110> BIOINCEPT, LLC.
<110> BARNEA, Eytan R.
<120> PIF Peptide Biologic Activities, site of Action, and the Antibody to Detect PIF
<130> 120785.00702
<140> not yet assigned
   <141> 2004-10-22
<150> 60/513,370 <151> 2003-10-22
<160> 17
<170> PatentIn version 3.2
<210> 1
   <211> 15
   <212> PRT
   <213> mammalian
   <220>
   <223> isolated native peptide nPIF-1[sub]15; matches region of circumsporozoite protein (malaria)
<400> 1
   Met Val Arg Ile Lys Pro Gly Ser Ala Asn Lys Pro Ser Asp Asp
   1 5 10 15
<210> 2
   <211> 15
   <212> PRT
   <213> mammalian
   <220>
   <223> isolated native peptide nPIF-1[sub]15-alter; matches region of circumsporozoite protein (malaria)
<400> 2
   Met Val Arg Ile Lys Tyr Gly Ser Tyr Asn Asn Lys Pro Ser Asp
   1               5                   10                  15
<210> 3
   <211> 13
   <212> PRT
   <213> mammalian
   <220>
   <223> isolated native peptide nPIF-1[sub]13; matches region of circumsporozoite protein (malaria)
<400> 3
   Met Val Arg Ile Lys Pro Gly Ser Ala Asn Lys Pro Ser
   1               5                   10
<210> 4
   <211> 9
   <212> PRT
   <213> mammalian
   <220>
   <223> isolated native peptide nPIF-1[sub]9; matches region of circumsporozoite protein (malaria)
<400> 4
   Met Val Arg Ile Lys Pro Gly Ser Ala
   1               5
<210> 5
   <211> 15
   <212> PRT
   <213> Artificial
<220>
   <223> synthetic peptide scrPIF-1[sub]15; scrambled amino acid sequence from region of circumsporozoite protein malaria
<400> 5
   Gly Arg Val Asp Pro Ser Asn Lys Ser Met Pro Lys Asp Ile Ala
   1               5                   10                  15
<210> 6
   <211> 10
   <212> PRT
   <213> Mammalian
   <220>
   <223> isolated native peptide nPIF-2[sub]10; matches region of human retinoid and thyroid hormone receptor (SMRT)
<400> 6
   Ser Gln Ala Val Gln Glu His Ala Ser Thr
   1 5 10
<210> 7
   <211> 13
   <212> PRT
   <213> mammalian
   <220>
   <223> isolated native peptide nPIF-2[sub]13; matches region of human retinoid and thyroid hormone receptor SMRT
<400> 7
   Ser Gln Ala Val Gln Glu His Ala Ser Thr Asn Met Gly
   1 5 10
<210> 8
   <211> 13
   <212> PRT
   <213> Artificial
<220>
   <223> synthetic peptide scrPIF-2[sub]13; scrambled amino acid sequence from region of human retinoid and thyroid hormone receptor SMRT
<400> 8
   Glu Val Ala Gln His Ser Gln Ala Ser Thr Met Asn Gly
   1 5 10
<210> 9
   <211> 14
   <212> PRT
   <213> Artificial
<220>
   <223> synthetic peptide scrPIF-2[sub]14; scrambled amino acid sequence from region of human retinoid and thyroid hormone receptor SMRT
<400> 9
   Gly Gln Ala Ser Ser Ala Gln Met Asn Ser Thr Gly Val His
   1 5 10
<210> 10
   <211> 18 <212> PRT
   <213> Mammalian
   <220>
   <223> isolated native peptide nPIF-3[sub]18; scrambled region of Rev Trans
<400> 10
   Ser Gly Ile Val Ile Tyr Gln Tyr Met Asp Asp Arg Tyr Val Gly Ser
   1 5 10 15
   Asp Leu
<210> 11
   <211> 11
   <212> PRT
   <213> Artificial
<220>
   <223> synthetic peptide Neg control for negPIF-1[sub]15; scrambled amino acid sequence from region of circumsporozoite protein malaria
<400> 11
   Gly Met Arg Glu Leu Gln Arg Ser Ala Asn Lys
   1 5 10
<210> 12
   <211> 9
   <212> PRT
   <213> mammalian
   <220>
   <223> isolated native peptide nPIF-4[sub]9; matches region of Rev Trans
<400> 12
   Val Ile Ile Ile Ala Gln Tyr Met Asp
   1 5
<210> 13
   <211> 15
   <212> PRT
   <213> Artificial
<220>
   <223> synthetic peptide sPIF-1[sub]15; amino acid sequence from region of circumsporozoite protein malaria
<400> 13
   Met Val Arg Ile Lys Pro Gly Ser Ala Asn Lys Pro Ser Asp Asp
   1 5 10 15
<210> 14
   <211> 13
   <212> PRT
   <213> Artificial
<220>
   <223> synthetic peptide sPIF-2[sub]13; amino acid sequence of human retinoid and thyroid hormone receptor SMRT
<400> 14
   Ser Gln Ala Val Gln Glu His Ala Ser Thr Asn Met Gly
   1               5                   10
<210> 15
   <211> 18
   <212> PRT
   <213> Artificial
<220>
   <223> synthetic peptide sPIF-3[sub]18; amino acid sequence from region of circumsporozoite protein malaria
<400> 15
   Ser Gly Ile Val Ile Tyr Gln Tyr Met Asp Asp Arg Tyr Val Gly Ser
   1               5                   10                  15
   Asp Leu
<210> 16
   <211> 9
   <212> PRT
   <213> Artificial
<220>
   <223> synthetic peptide sPIF-1[sub]9; amino acid sequence from region of circumsporozoite protein malaria
<400> 16
   Met Val Arg Ile Lys Pro Gly Ser Ala
   1               5
<210> 17
   <211> 9
   <212> PRT
   <213> Artificial
<220>
   <223> synthetic peptide sPIF-4[sub]9
<400> 17
   Val Ile Ile Ile Ala Gln Tyr Met Asp
   1 5